# EUROPEAN PATENT SPECIFICATION

(11) **EP 3 294 897 B1**
(45) Date of publication and mention of the grant of the patent: **04.09.2024**
(21) Application number: 16728840.6
(22) Date of filing: 12.05.2016
(51) Int. Cl.: C12P 7/22, C12N 15/81

(54) **A METHOD FOR PRODUCING RESVERATROL**
VERFAHREN ZUR GEWINNUNG VON RESVERATROL
PROCÉDÉ POUR LA PRODUCTION DE RESVÉRATROL

(30) Priority: 12.05.2015 US 201562160505 P
(43) Date of publication of application: 21.03.2018
(73) Proprietor: Danstar Ferment AG, 6300 Zug (CH)
(72) Inventor: SIMON VECILLA, Ernesto, DK-2600 Glostrup (DK); VAZQUEZ, Carlos, Casado, DK-2200 Copenhagen (DK); JENSEN, Niels, Bjerg, CH-4153 Reinach (CH)
(74) Representative: Forrest, Stuart
(86) International application number: PCT/EP2016/060798
(87) International publication number: WO 2016/180956

(56) References cited:
- WO-A1-2006/089898
- WO-A2-2011/147818
- LUTTIK M A H ET AL: "Alleviation of feedback inhibition in Saccharomyces cerevisiae aromatic amino acid biosynthesis: Quantification of metabolic impact", METABOLIC ENGINEERING, ACADEMIC PRESS, US, vol. 10, no. 3-4, 1 May 2008 (2008-05-01), pages 141 - 153, XP022715254, ISSN: 1096-7176, [retrieved on 20080220], DOI: 10.1016/J.YMBEN.2008.02.002
- SCHMIDHEINI T ET AL: "SINGLE POINT MUTATION RESULTS IN A CONSTITUTIVELY ACTIVATED AND FEEDBACK-RESISTANT CHORISMATE MUTASE OF SACCHAROMYCES CEREVISIAE", JOURNAL OF BACTERIOLOGY, AMERICAN SOCIETY FOR MICROBIOLOGY, US, vol. 171, no. 3, 1 March 1989 (1989-03-01), pages 1245 - 1253, XP000946041, ISSN: 0021-9193
- S. SHI ET AL: "Improving Production of Malonyl Coenzyme A-Derived Metabolites by Abolishing Snf1-Dependent Regulation of Acc1", MBIO, vol. 5, no. 3, 6 May 2014 (2014-05-06), pages e01130 - 14, XP055289711, DOI: 10.1128/mBio.01130-14
- MINGJI LI ET AL: "De novo production of resveratrol from glucose or ethanol by engineered Saccharomyces cerevisiae", METABOLIC ENGINEERING, vol. 32, 1 November 2015 (2015-11-01), US, pages 1 - 11, XP055289397, ISSN: 1096-7176, DOI: 10.1016/j.ymben.2015.08.007

## Description

### BACKGROUND OF THE INVENTION

### Field of the Invention

The invention disclosed herein relates generally to the fields of genetic engineering. Particularly, the invention disclosed herein provides methods for producing increased yields of stilbenes and flavonoids in a genetically modified cell and to recombinant host microorganism cells capable of producing a stilbene.

### Description of Related Art

Flavonoids and stilbenes are two groups of secondary plant metabolites derived from the phenylpropanoid pathway by different routes. Both flavonoids and stilbenes are recognized as having antifungal and antibacterial properties in plants. Flavonoids have also been investigated for application to several human pathological conditions, including cancer, diabetes, obesity and Parkinson's disease (see *e.g.,* Koopman et al., 2012, Microbial Cell Factories 11:155; Wang & Zhang, 2012, Scanning 34: 1-5; Zava & Duwe, 1997, Nutr. Cancer 27: 31-40; Greenwald, 2004, J Nutr 134: 3507S-3512S; Hou et al., 2004, J Biomed Biotechnol 2004: 321-325; Allister et al., 2005, Diabetes 54: 1676-1683). Flavonoids have been shown to act as scavengers of oxygen radicals, and may possess anti-inflammatory, antiviral and anti-tumor activities (*see e.g.*, Koopman et al., 2012, Microbial Cell Factories 11:155; Nijveldt et al., 2001, Am J Clin Nutr 74: 418-425; Limem et al., 2008, Process Biochem 43:463-479). Currently, flavonoid production is mainly achieved by isolation from plants. Production of flavonoids is inefficient, however, due primarily to low growth rate of producing plants coupled with complex extraction and separation of flavonoids from related structures.

Resveratrol (3,5,4'-trihydroxy-stilbene) is a phytophenol belonging to the group of stilbene phytoalexins, which are low-molecular-mass secondary metabolites that constitute the active defense mechanism in plants in response to fungal and other infections or other stress-related events (*see*, *e.g.*, WO2006089898 (A1)). In addition to its antifungal properties, resveratrol has been recognized for its cardioprotective and cancer chemopreventive activities in humans; it acts as a phytoestrogen, an inhibitor of platelet aggregation (Kopp et al., 1998, European J Endocrinol. 138: 619-620; Gehm et al., 1997, Proc Natl Acad Sci USA 94: 14138-14143; Lobo et al., 1995, Am. J. Obstet. Gynecol. 173: 982-989; Gao & Ming, 2010, Mini Rev Med Chem 10(6):550-67), and an antioxidant (Tang et al., 1997, Science 275: 218-220; Huang, 1997, Food Sci. 24: 713-727).

Plants, the skin of red grapes, and other fruits produce resveratrol naturally. Present production processes rely mostly upon extraction of resveratrol, either from the skin of grape berries or from the plant *Fallopia japonica* or *Polygonum cuspidatum*, known as "Japanese knotweed." Current extraction and purification methods use organic solvents to extract resveratrol and separate it from the biomass and/or cell debris. Examples of these solvents include, among others, ethanol, methanol, ethyl acetate, and petroleum ether. This is a labor-intensive process and generates low yields. Moreover, since resveratrol has low water-solubility (*see*, *e.g.*, Gao & Ming, 2010, Mini Rev Med Chem 10(6):550-67), it forms aggregates/crystals upon addition to water and/or formation by a recombinant resveratrol producing- and secreting-microorganism. Separation of resveratrol aggregates/crystals from recombinant or other cells (such as microorganisms or plant cells) by centrifugation is inefficient.

Generally, stilbenes, including resveratrol, and flavonoids are produced in plants and yeast through the phenylpropanoid pathway as illustrated by the reactions shown in Figures 1 and 2 and as described in WO2006089898 (A1). In yeast, the starting metabolites are malonyl-CoA and phenylalanine or tyrosine. The amino acid L-phenylalanine is converted into trans-cinnamic acid through non-oxidative deamination by L-phenylalanine ammonia lyase (PAL). Next, trans-cinnamic acid is hydroxylated at the para-position to 4-coumaric acid (4-hydroxycinnamic acid) by cinnamate-4-hydroxylase (C4H), a cytochrome P450 monooxygenase enzyme, in conjunction with NADPH:cytochrome P450 reductase (CPR). Alternatively, the amino acid L-tyrosine is converted into 4-coumaric acid by tyrosine ammonia lyase (TAL). The 4-coumaric acid from either alternative pathway is subsequently activated to 4-coumaroyl-CoA by the action of 4-coumarate-CoA ligase (4CL). Within the phenylpropanoid pathway, 4-coumaroyl-CoA represents the key branching point from which flavonoids and stilbenes are derived. Stilbenes are synthesized via stilbene synthase (STS), also known as resveratrol synthase (RS), catalyzing condensation of a phenylpropane unit of 4-coumaroyl-CoA with malonyl-CoA, resulting in formation of resveratrol. Conversely, the first step in flavonoid synthesis is condensation of a phenylpropane unit of 4-coumaroyl-CoA with malonyl-CoA and chalcone synthase (CHS), resulting in the formation of tetrahydroxychalcone.

Previously, a yeast strain was disclosed that could produce resveratrol from 4-coumaric acid that is found in small quantities in grape must (Becker et al., 2003, FEMS Yeast Res. 4: 79-85). Production of 4-coumaroyl-CoA, and concomitantly resveratrol, in laboratory strains of *Saccharomyces cerevisiae,* has been achieved by co-expressing a heterologous coenzyme-A ligase gene from hybrid poplar, together with the grapevine resveratrol synthase gene (*vst1*) (Becker et al., 2003, FEMS Yeast Res. 4:79-85). Another substrate for resveratrol synthase, malonyl-CoA, is endogenously produced in yeast. Becker *et al.*, 2003, ld., indicated that *S*. *cerevisiae* cells produced minute amounts of resveratrol in the piceid form when cultured in synthetic media supplemented with 4-coumaric acid. However, said yeast strain would not be suitable for commercial application because it results in low resveratrol yields and requires the addition of 4-coumaric acid, which is expensive and not often present in industrial media. Therefore, there remains a need for an *in vivo* expression system that produces high yields of resveratrol more economically.

### SUMMARY OF THE INVENTION

It is against the above background that the present invention provides certain advantages and advancements over the prior art.

Although this invention disclosed herein is not limited to specific advantages or functionality, the invention disclosed herein provides a recombinant host comprising:
(a) a gene encoding a tyrosine-sensitive 3-Deoxy-D-arabinoheptulosonate 7-phospate synthase (ARO4) polypeptide having at least 65% identity to the amino acid sequence set forth in SEQ ID NO: 1,
   wherein the ARO4 polypeptide comprises a mutation that is K229L and is not feedback inhibited;
(b) a gene encoding a chorismate mutase polypeptide;
(c) a gene encoding an acetyl-CoA carboxylase polypeptide; and
(d) disruption of a gene encoding a phenylalanine-inhibited 3-Deoxy-D-arabinoheptulosonate 7-phosphate synthase (ARO3) polypeptide having at least 75% identity to the amino acid sequence set forth in SEQ ID NO: 3, whereby the host does not express ARO3;

wherein at least one of the genes is a recombinant gene,
wherein the host is capable of producing a stilbene.

The invention further provides a method of producing a stilbene, comprising:
(a) growing a recombinant host microorganism in a culture medium, under conditions in which the genes are expressed,
   wherein the host comprises:
      (i) a gene encoding a tyrosine-sensitive 3-Deoxy-D-arabinoheptulosonate 7-phosphate synthase (ARO4) polypeptide having at least 65% identity to the amino acid sequence set forth in SEQ ID NO:1, wherein the ARO4 polypeptide comprises a mutation that is K229L and is not feedback inhibited;
      (ii) a gene encoding a chorismate mutase polypeptide;
      (iii) a gene encoding an acetyl-CoA carboxylase polypeptide; and
      (iv) disruption of a gene encoding a phenylalanine-inhibited 3-Deoxy-D-arabinoheptulosonate 7-phosphate synthase (ARO3) polypeptide having at least 75% identity to the amino acid sequence set forth in SEQ ID NO: 3, whereby the host does not express ARO3;
   wherein at least one of the genes is a recombinant gene,
   and, optionally
(b) recovering the stilbene from the culture media.

in some aspects, the chorismate mutase polypeptide comprises a chorismate mutase (ARO7) polypeptide having at least 50% identity to the amino acid sequence set forth in SEQ ID NO: 4.

In some aspects, the chorismate mutase polypeptide comprises an ARO7 polypeptide having a mutation at T226.

in some aspects, the chorismate mutase polypeptide comprises an ARO7 polypeptide having a mutation that is T226I or T226N.

in some aspects, the chorismate mutase polypeptide is not feedback inhibited.

In some aspects, the acetyl-CoA carboxylase polypeptide comprises an acetyl-CoA carboxylase alpha (ACC1) polypeptide having at least 70% identity to the amino acid sequence set forth in SEQ ID NO: 6.

in some aspects, the acetyl-CoA carboxylase polypeptide comprises an ACC1 polypeptide having at least one mutation at S659 or S1157.

in some aspects, the acetyl-CoA carboxylase polypeptide comprises an ACC1 polypeptide having at least one mutation at S659 or S1157, wherein the replacement amino acid is non-phosphorylable.

in some aspects, the acetyl-CoA carboxylase polypeptide comprises an ACC1 polypeptide having at least one mutation that is S659A, S659V, S1157A, or S1157V.

in some aspects, the acetyl-CoA carboxylase polypeptide is not feedback inhibited.

in some embodiments of the recombinant host or methods disclosed herein, the recombinant host further comprises one or more of:
(a) a gene encoding a L-phenylalanine ammonia lyase (PAL) polypeptide;
(b) a gene encoding a cinnamate-4-hydroxylase (C4H) polypeptide;
(c) a gene encoding a NADPH:cytochrome P450 reductase polypeptide;
(d) a gene encoding a tyrosine ammonia lyase (TAL) polypeptide;
(e) a gene encoding a 4-coumarate-CoA ligase (4CL) polypeptide; or
(f) a gene encoding a stilbene synthase (STS) polypeptide;
wherein at least one of the genes is a recombinant gene.

In some aspects of the recombinant host or methods disclosed herein, the stilbene produced by the recombinant host is resveratrol or a resveratrol derivative.

In some aspects of the recombinant host or methods disclosed herein, the recombinant host comprises a microorganism that is a yeast cell, a plant cell, a mammalian cell, an insect cell, a fungal cell, or a bacterial cell.

In some aspects of the recombinant host or methods disclosed herein, the bacterial cell comprises *Escherichia* bacteria cells, *Lactobacillus* bacteria cells, *Lactococcus* bacteria cells, *Cornebacterium* bacteria cells, *Acetobacter* bacteria cells, *Acinetobacter* bacteria cells, or *Pseudomonas* bacterial cells.

In some aspects of the recombinant host or methods disclosed herein, the yeast cell is a cell from *Saccharomyces cerevisiae*, *Schizosaccharomyces pombe*, *Yarrowia lipolytica*, *Candida glabrata, Ashbya gossypii*, *Cyberlindnera jadinii, Pichia pastoris, Kluyveromyces lactis, Hansenula polymorpha, Candida boidinii, Arxula adeninivorans*, *Xanthophyllomyces dendrorhous,* or *Candida albicans* species.

In some aspects of the recombinant host or methods disclosed herein, the yeast cell is a *Saccharomycete.*

In some aspects of the recombinant host or methods disclosed herein, the yeast cell is a cell from the *Saccharomyces cerevisiae* species.

In some aspects, the methods disclosed herein further comprise the step of detecting the recovered stilbene by thin layer chromatography (TLC), high-performance liquid chromatography (HPLC), ultraviolet-visible spectroscopy/spectrophotometry (UV-Vis), liquid chromatography-mass spectrometry (LC-MS), or nuclear magnetic resonance (NMR).

Also disclosed herein but not claimed is a recombinant host comprising one or more of:
(a) a gene encoding a 3-Deoxy-D-arabinoheptulosonate 7-phosphate (DAHP) synthase polypeptide;
(b) a gene encoding a chorismate mutase polypeptide; and
(c) a gene encoding an acetyl-CoA carboxylase polypeptide;

wherein at least one of the genes is a recombinant gene,
wherein the host is capable of producing a flavonoid compound.

Also disclosed herein but not claimed is a method of producing a flavonoid compound, comprising:
(a) growing a recombinant host in a culture medium, under conditions in which the host produces a flavonoid,
   wherein the host comprises one or more of:
      (i) a gene encoding a 3-Deoxy-D-arabinoheptulosonate 7-phosphate (DAHP) synthase polypeptide;
      (ii) a gene encoding a chorismate mutase polypeptide; and
      (iii) a gene encoding an acetyl-CoA carboxylase polypeptide;
   wherein at least one of the genes is a recombinant gene,
   and, optionally
(b) recovering the flavonoid from the culture media.

In the recombinant host or methods disclosed, optionally the recombinant host further comprises one or more of:
(a) a gene encoding a L-phenylalanine ammonia lyase (PAL) polypeptide;
(b) a gene encoding a cinnamate-4-hydroxylase (C4H) polypeptide;
(c) a gene encoding a NADPH:cytochrome P450 reductase polypeptide;
(d) a gene encoding a tyrosine ammonia lyase (TAL) polypeptide;
(e) a gene encoding a 4-coumarate-CoA ligase (4CL) polypeptide; or
(f) a gene encoding a chalcone synthase (CHS) polypeptide;
wherein at least one of said genes is a recombinant gene.

These and other features and advantages of the present invention will be more fully understood from the following detailed description of the invention taken together with the accompanying claims. It is noted that the scope of the claims is defined by the recitations therein and not by the specific discussion of features and advantages set forth in the present description.

### BRIEF DESCRIPTION OF THE DRAWINGS

The following detailed description of the embodiments of the present invention can be best understood when read in conjunction with the following drawings, where like structure is indicated with like reference numerals and in which:
Figure 1 shows a schematic diagram of the resveratrol pathway from L-phenylalanine or L-tyrosine in plants and yeast.
Figure 2 shows a schematic diagram of a pathway for producing resveratrol from glucose in yeast.

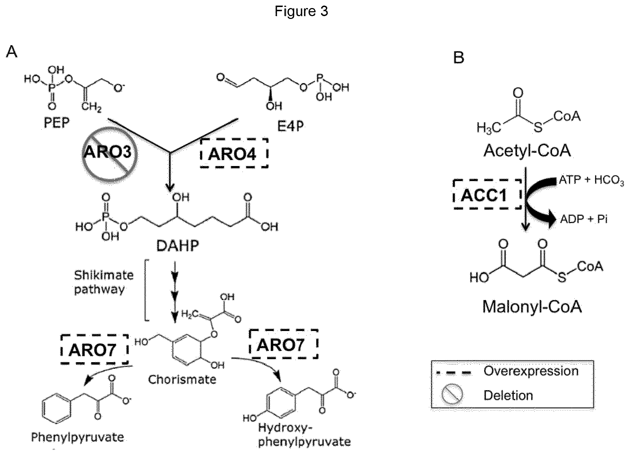

Figure 3A shows a schematic diagram of a pathway for producing phenylpyruvate and hydroxyphenipyruvate from phosphoenolpyruvate and erythrose 4-phosphate in an S. *cerevisiae* strain overexpressing tyrosine-sensitive 3-Deoxy-D-arabinoheptulosonate 7-phosphate synthase (ARO4) and chorismate mutase (ARO7) and deleted of phenylalanine-inhibited 3-Deoxy-D-arabinoheptulosonate 7-phosphate synthase (ARO3).
Figure 3B shows a schematic diagram of a pathway for producing malonyl-CoA from acetyl-CoA in an *S*. *cerevisiae* strain overexpressing acetyl-CoA carboxylase alpha (ACC1).

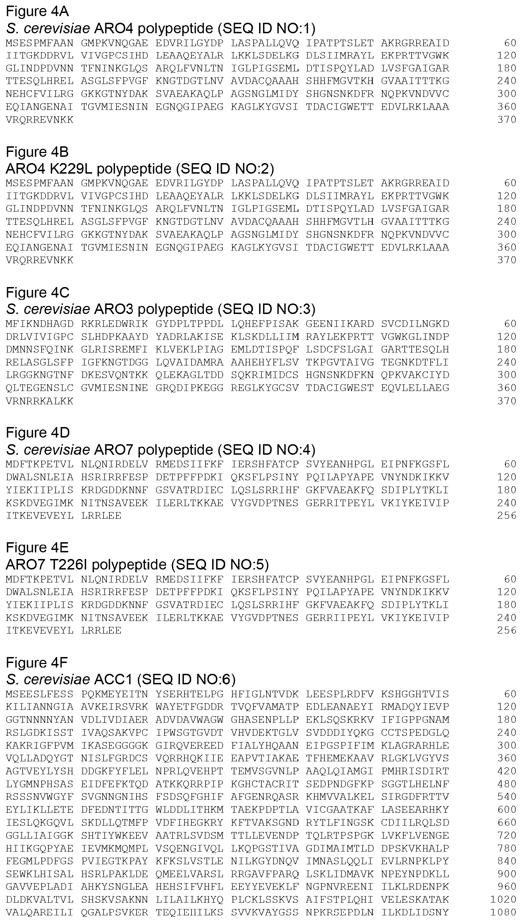

Figure 4A shows the amino acid sequence of *S*. *cerevisiae* ARO4 (SEQ ID NO:1).
Figure 4B shows amino acid sequence of the K229L mutant of *S*. *cerevisiae* ARO4 (SEQ ID NO:2).
Figure 4C shows the amino acid sequence of *S*. *cerevisiae* ARO3 (SEQ ID NO:3).
Figure 4D shows the amino acid sequence of *S*. *cerevisiae* ARO7 (SEQ ID NO:4).
Figure 4E shows the amino acid sequence of the T226I mutant of *S*. *cerevisiae* ARO7 (SEQ ID NO:5).
Figure 4F shows *S*. *cerevisiae* ACC1 (SEQ ID NO:6).

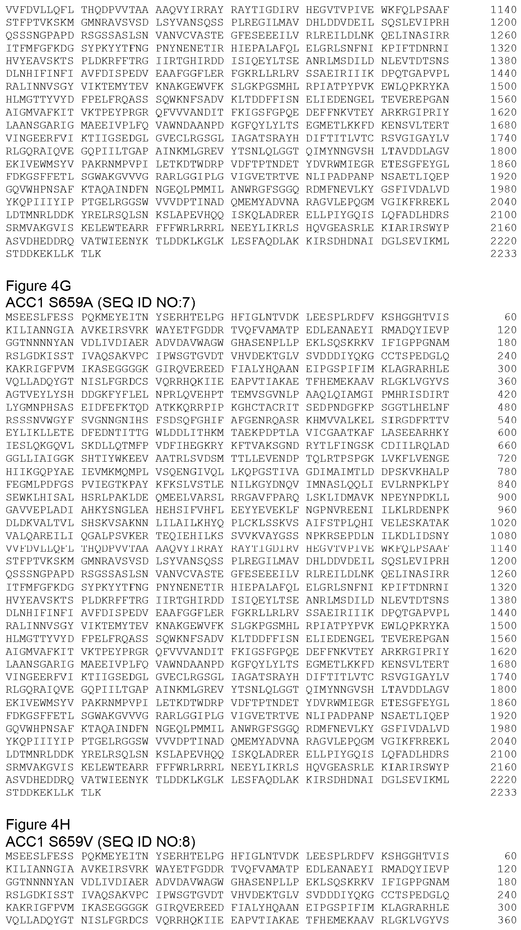

Figure 4G shows the amino acid sequence of the S659A mutant of *S*. *cerevisiae* ACC1 (SEQ ID NO:7).
Figure 4H shows the amino acid sequence of the S659V mutant of *S*. *cerevisiae* ACC1 (SEQ ID NO:8).

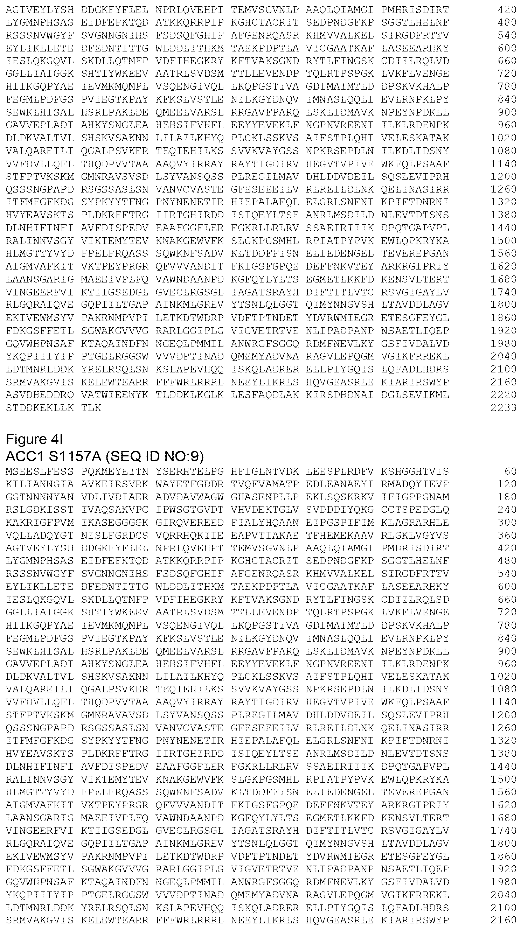

Figure 4I shows the amino acid sequence of the S1157A mutant of *S*. *cerevisiae* ACC1 (SEQ ID NO:9).

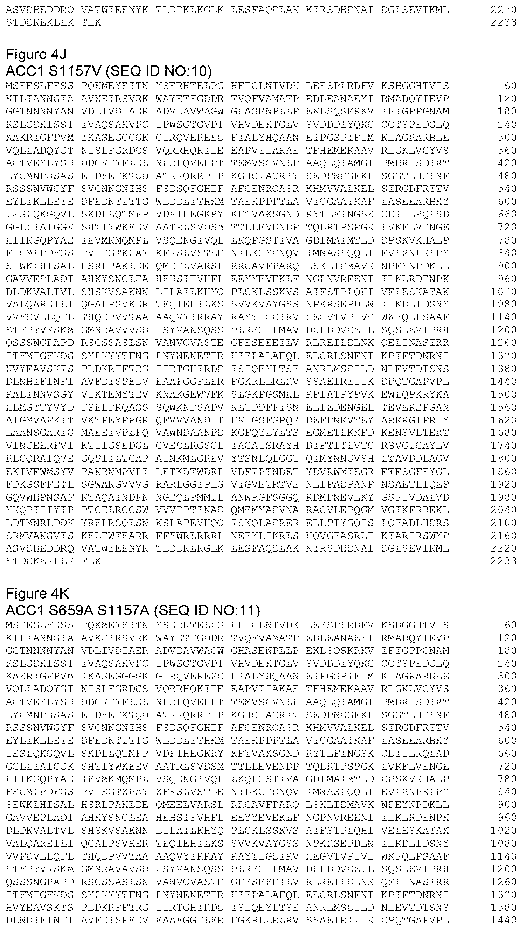

Figure 4J shows the amino acid sequence of the S1157V mutant of *S*. *cerevisiae* ACC1 (SEQ ID NO:10).
Figure 4K shows the amino acid sequence of the S659A S1157A mutant of *S*. *cerevisiae* ACC1 (SEQ ID NO:11).

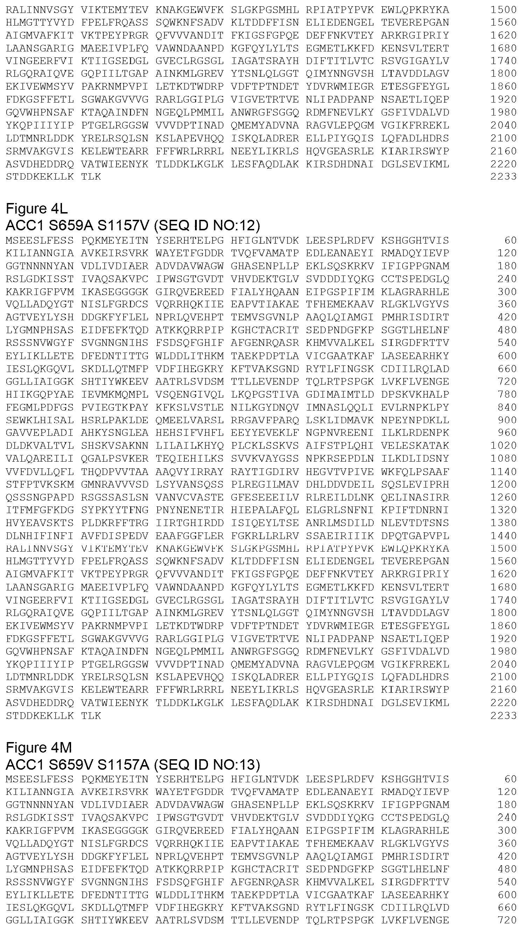

Figure 4L shows the amino acid sequence of the S659A S1157V mutant of *S*. *cerevisiae* ACC1 (SEQ ID NO:12).
Figure 4M shows the amino acid sequence of the S659V S1157A mutant of *S*. *cerevisiae* ACC1 (SEQ ID NO:13).

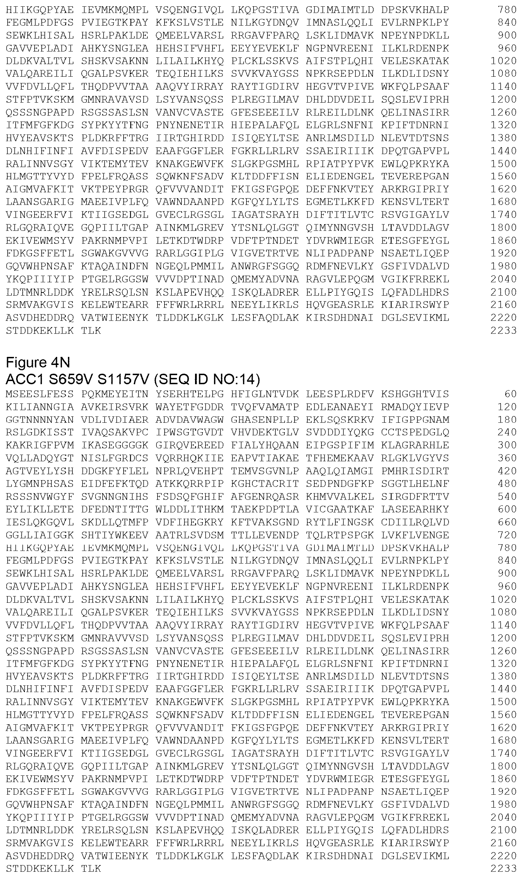

Figure 4N shows the amino acid sequence of the S659V S1157V mutant of *S*. *cerevisiae* ACC1 (SEQ ID NO:14).

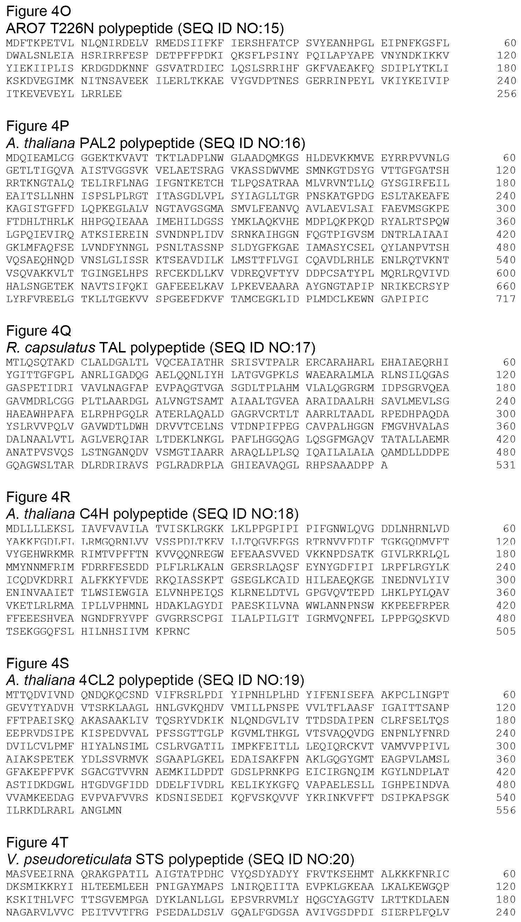

Figure 4O shows the amino acid sequence of the T226N mutant of *S*. *cerevisiae* ARO7 (SEQ ID NO:15).
Figure 4P shows the amino acid sequence of *A. thaliana* PAL2 (SEQ ID NO:16).
Figure 4Q shows the amino acid sequence of *R*. *capsulatus* TAL (SEQ ID NO:17).
Figure 4R shows the amino acid sequence of *A*. *thaliana* C4H (SEQ ID NO:18).
Figure 4S shows the amino acid sequence of *A*. *thaliana* 4CL2 (SEQ ID NO:19).
Figure 4T shows the amino acid sequence of *V*. *pseudoreticulata* STS (SEQ ID NO:20).

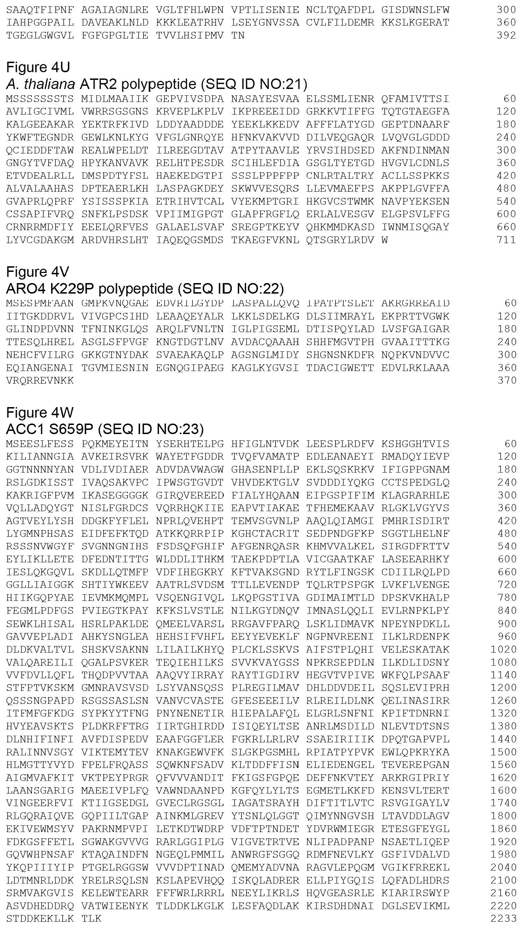

Figure 4U shows the amino acid sequence of *A*. *thaliana* ATR2 (SEQ ID NO:21).
Figure 4V shows the amino acid sequence of the K229P mutant of *S*. *cerevisiae* ARO4 (SEQ ID NO:22).
Figure 4W shows the amino acid sequence of the S659P mutant of *S*. *cerevisiae* ACC1 (SEQ ID NO:23).

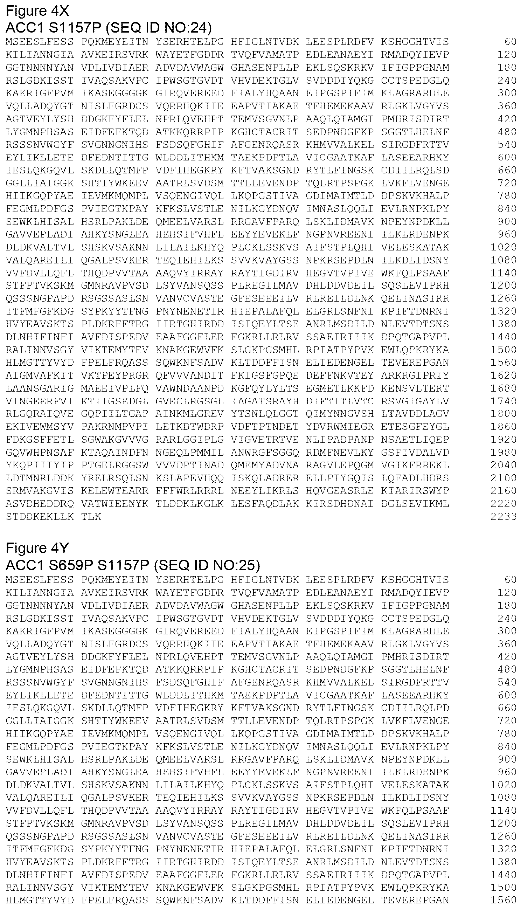

Figure 4X shows the amino acid sequence of the S1157P mutant of *S. cerevisiae* ACC1 (SEQ ID NO:24).
Figure 4Y shows the amino acid sequence of the S659P S1157P mutant of *S*. *cerevisiae* ACC1 (SEQ ID NO:25).

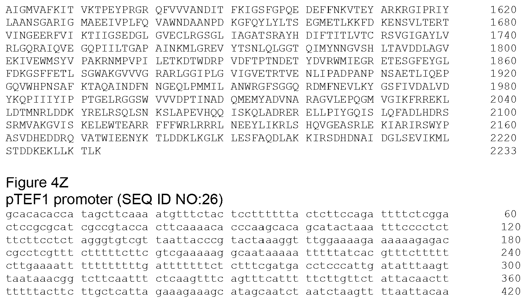

Figure 4Z shows the nucleic acid sequence of the pTEF1 promoter (SEQ ID NO:26).

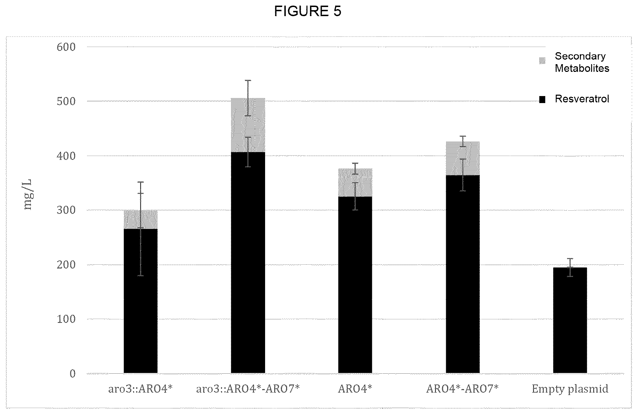

Figure 5 shows production of resveratrol and secondary metabolites in a yeast strain transformed with empty plasmid (control strain), a yeast strain overexpressing mutant *S*. *cerevisiae* ARO4 K229L and ARO7 T226I genes (ARO4*-ARO7*), a yeast strain overexpressing mutant *S*. *cerevisiae ARO4* K229L gene (ARO4*), an *ARO3* knockout yeast strain overexpressing mutant *S*. *cerevisiae ARO4* K229L and *ARO7* T226I genes (aro3::ARO4*-ARO7*), and an *ARO3* knockout yeast strain overexpressing mutant S. *cerevisiae ARO4* K229L gene (aro3::ARO4*). Secondary metabolites are likely produced due to conversion of starting metabolites malonyl-CoA, phenylalanine and/or tyrosine into intermediates (*e*.*g*., coumaric acid) and side products (*e*.*g*., phloretic acid), rather than resveratrol.
Figure 6 shows resveratrol titers over time of fed-batch fermentation in an *ARO3* knockout yeast strain overexpressing mutant *S*. *cerevisiae ARO4* K229L and *ARO7* T226I genes (aro3::ARO4*-ARO7*) and a reference strain in which the same strain above (aro3::ARO4*-ARO7*) was further transformed with a URA3 selection marker deleting the overexpressed *ARO4* K229L and *ARO7* T226I genes (aro3::URA3).

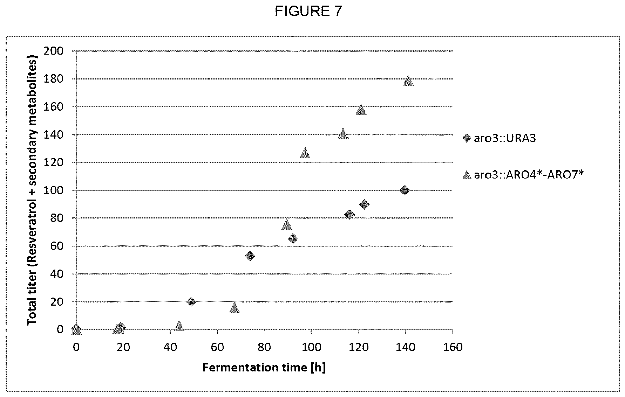

Figure 7 shows total titer of resveratrol and secondary metabolites over time of fed-batch fermentation in an *ARO3* knockout yeast strain overexpressing mutant S. *cerevisiae ARO4* K229L and *ARO7* T226I genes (aro3::ARO4*-ARO7*) and a reference strain in which the same strain above (aro3::ARO4*-ARO7*) was further transformed with a URA3 selection marker deleting the overexpressed *ARO4* K229L and *ARO7* T226I genes (aro3::URA3). Secondary metabolites are likely produced due to conversion of starting metabolites malonyl-CoA, phenylalanine and/or tyrosine into intermediates (*e.g*., coumaric acid) and side products (*e.g*., phloretic acid), rather than resveratrol.
Figure 8 shows resveratrol titer during fed-batch cultivation of resveratrol-producing strains. Titers are expressed as improvement relative to the titer at 140 hours of the strain overexpressing only the endogenous, not mutated, *ACC1* due to promoter replacement with the strong constitutive promoter pTEF1.

Skilled artisans will appreciate that elements in the Figures are illustrated for simplicity and clarity and have not necessarily been drawn to scale. For example, the dimensions of some of the elements in the Figures can be exaggerated relative to other elements to help improve understanding of the embodiment(s) of the present invention.

### DETAILED DESCRIPTION OF THE INVENTION

Methods well known to those skilled in the art can be used to construct genetic expression constructs and recombinant cells according to this invention. These methods include *in vitro* recombinant DNA techniques, synthetic techniques, *in vivo* recombination techniques, and PCR techniques. See, *for example*, techniques as described in Maniatis et al., 1989, MOLECULAR CLONING: A LABORATORY MANUAL, Cold Spring Harbor Laboratory, New York; Ausubel et al., 1989, CURRENT PROTOCOLS IN MOLECULAR BIOLOGY, Greene Publishing Associates and Wiley Interscience, New York, and PCR Protocols: A Guide to Methods and Applications (Innis et al., 1990, Academic Press, San Diego, CA).

Before describing the present invention in detail, a number of terms will be defined. As used herein, the singular forms "a", "an", and "the" include plural referents unless the context clearly dictates otherwise. For example, reference to a "nucleic acid" means one or more nucleic acids.

It is noted that terms like "preferably', "commonly", and "typically" are not utilized herein to limit the scope of the claimed invention or to imply that certain features are critical, essential, or even important to the structure or function of the claimed invention. Rather, these terms are merely intended to highlight alternative or additional features that can or cannot be utilized in a particular embodiment of the present invention.

For the purposes of describing and defining the present invention it is noted that the terms "increase", "increases", "increased", "greater", 'higher", and "lower" are utilized herein to represent non-quantitative comparisons, values, measurements, or other representations to a stated reference or control.

For the purposes of describing and defining the present invention it is noted that the term "substantially" is utilized herein to represent the inherent degree of uncertainty that can be attributed to any quantitative comparison, value, measurement, or other representation. The term "substantially" is also utilized herein to represent the degree by which a quantitative representation can vary from a stated reference without resulting in a change in the basic function of the subject matter at issue.

As used herein, the terms "polynucleotide", "nucleotide", "oligonucleotide", and "nucleic acid" can be used interchangeably to refer to nucleic acid comprising DNA, RNA, derivatives thereof, or combinations thereof.

As used herein, the terms "feedback-inhibited" and "feedback inhibited" are used interchangeably to refer to deactivation of a stated enzyme by a product of the reaction catalyzed by the same enzyme once the product reaches a threshold level.

As used herein, the term "tyrosine-sensitive" refers to an enzyme which can be feedback-inhibited by tyrosine.

### Production of Resveratrol or Modified Resveratrol

Resveratrol can be synthesized *in vitro,* by bioconversion, or in a recombinant host. As used herein, the term "recombinant host" is intended to refer to a host cell, the genome of which has been augmented by at least one incorporated DNA sequence. Such DNA sequences include, but are not limited to, genes that are not naturally present, DNA sequences that are not normally transcribed into RNA or translated into a protein ("expressed"), and other genes or DNA sequences that are desired to be introduced into the cell to produce the recombinant host. It will be appreciated that the genome of a recombinant host described herein is typically augmented through stable introduction of one or more recombinant genes. Generally, the introduced DNA is not originally resident in the host that is the recipient of the DNA, but it is within the scope of the invention to isolate a DNA segment from a given host, and to subsequently introduce one or more additional copies of that DNA into the same host, *e*.*g*., to enhance production of the product of a gene or alter the expression pattern of a gene. In some instances, the introduced DNA will modify or replace an endogenous gene or DNA sequence by, *e*.*g*., homologous recombination or site-directed mutagenesis. Suitable recombinant hosts include microorganisms, plant cells, and plants.

The term "recombinant gene" refers to a gene or DNA sequence that is introduced into a recipient host, regardless of whether the same or a similar gene or DNA sequence may already be present in such a host. "Introduced" or "augmented" in this context is known in the art to mean introduced or augmented by the hand of man. Thus, a recombinant gene may be a DNA sequence from another species, or may be a DNA sequence that originated from or is present in the same species, but has been incorporated into a host by recombinant methods to form a recombinant host. It will be appreciated that a recombinant gene that is introduced into a host can be identical to a DNA sequence that is normally present in the host being transformed and is introduced to provide one or more additional copies of the DNA to thereby permit overexpression or modified expression of the gene product of that DNA. In a preferred embodiment, the DNA is a cDNA copy of an mRNA transcript of a gene produced in a cell. As used herein, the terms "codon optimization" and "codon optimized" refers to a technique to maximize protein expression in fast-growing microorganisms such as *Escherichia coli* or *Saccharomyces cerevisiae* by increasing the translation efficiency of a particular gene. Codon optimization can be achieved, *for example*, by transforming nucleotide sequences of one species into the genetic sequence of a different species. Optimal codons help to achieve faster translation rates and high accuracy. As a result of these factors, translational selection is expected to be stronger in highly expressed genes.

As used herein, "reduced expression" refers to expression of a gene or protein at a level lower than the native expression of the gene or protein. For example, in some embodiments the activity of a reductase is reduced by decreasing the amount of protein product, or expression, of a gene encoding the reductase.

Reduction or elimination (*i*.*e*., disruption) of expression of a gene can be accomplished by any known method, including insertions, missense mutations, frame shift mutations, deletion, substitutions, or replacement of a DNA sequence, or any combinations thereof. Insertions include the insertion of the entire genes, which may be of any origin. Reduction or elimination of gene expression can, for example, comprise altering or replacing a promoter, an enhancer, or splice site of a gene, leading to inhibition of production of the normal gene product partially or completely. In some embodiments, reduction or elimination of gene expression comprises altering the total level of the protein product expressed in the cell or organism. In other embodiments, disruption of a gene comprises reducing or eliminating the activity of the protein product of the gene in a cell or organism. In some embodiments of the disclosure, the disruption is a null disruption, wherein there is no significant expression of the gene. In some embodiments the disruption of a gene in a host cell or organism occurs on both chromosomes, in which case it is a homozygous disruption. In other embodiments the disruption of a gene in a host cell or organism occurs on only one chromosome, leaving the other chromosomal copy intact, in which case it is a heterozygous gene disruption. In still other embodiments each copy of a gene in a host cell or organism is disrupted differently.

Reduction or elimination of gene expression may also comprise gene knock-out or knock-down. A "gene knock-out" refers to a cell or organism in which the expression of one or more genes is eliminated, and therefore the cell or organism does not express the one or more genes knocked-out. A "gene knock-down" refers to a cell or organism in which the level of one or more genes is reduced, but not completely eliminated.

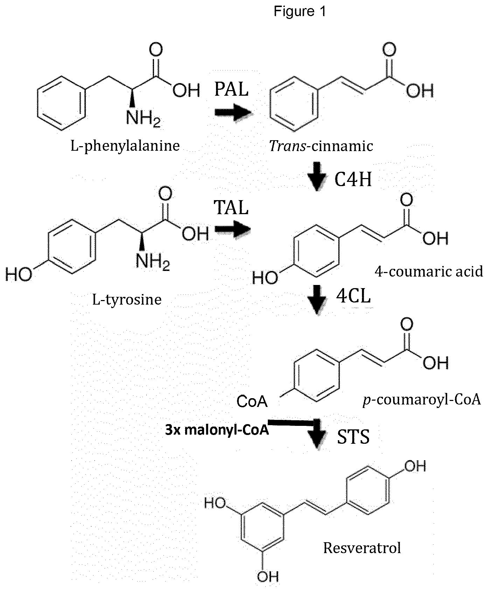

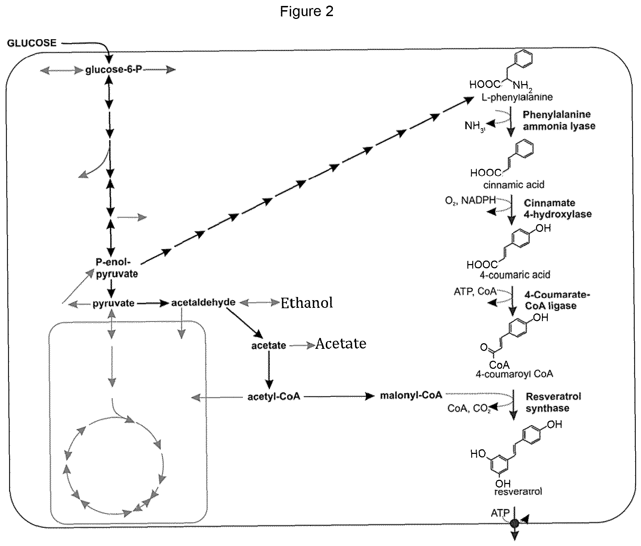

As used herein, the terms "resveratrol-producing strain," "resveratrol-producing cells," "resveratrol-producing host," and "resveratrol-producing microorganism" can be used interchangeably to refer to cells that express genes encoding proteins involved in resveratrol production (*see*, *e.g.*, Figures 1, 2). For example, a resveratrol-producing strain can express genes encoding one or more of an L-phenylalanine ammonia lyase (PAL) polypeptide, a cinnamate-4-hydroxylase (C4H) polypeptide, a cytochrome P450 monooxygenase polypeptide, an NADPH:cytochrome P450 reductase polypeptide, a 4-coumarate-CoA ligase (4CL) polypeptide, and a stilbene synthase (STS) polypeptide. In another example, a resveratrol-producing strain can express genes encoding one or more of a tyrosine ammonia lyase (TAL), a 4-coumarate-CoA ligase (4CL) polypeptide, and a stilbene synthase (STS) polypeptide. One or more of the genes encoding proteins involved in resveratrol production can be recombinant. *See*, *e.g.*, WO2006/089898, WO2008/009728, WO2009/016108, WO2009/124879, WO2009/124967, WO2011/147818,

Optionally, a stilbene-producing host comprises a gene encoding a4-coumarate-CoA ligase (4CL) polypeptide and a gene encoding stilbene synthase (STS) polypeptide, wherein the host is capable of producing the stilbene from a carbon source when the host is fed, *for example*, but not limited to, coumaric acid. *See*, *e.g.*, Wang et al., Annals of Microbiology, 2014, tSSN 1590-4261.

in some embodiments, a L-phenylalanine ammonia lyase (PAL) polypeptide can be expressed, overexpressed, or recombinantly expressed in said microorganism. Said PAL may optionally be a PAL (EC 4.3.1.5) from a plant belonging to the genus of *Arabidopsis, Brassica, Citrus, Phaseolus, Pinus, Populus*, *Solanum, Prunus, Vitis*, *Zea, Agastache, Ananas, Asparagus, Bromheadia, Bambusa, Beta, Betula, Cucumis*, *Camellia, Capsicum, Cassia, Catharanthus*, *Cicer*, *Citrultus, Coffea, Cucurbita*, *Cynodon, Daucus, Dendrobium*, *Dianthus, Digitalis, Dioscorea, Eucalyptus, Gallus, Ginkgo, Glycine, Hordeum, Helianthus, Ipomoea, Lactuca, Lithospermum, Lotus, Lycopersicon, Medicago, Malus, Manihot, Medicago, Mesembryanthemum, Nicotiana, Olea, Oryza, Pisum, Persea, Petroselinum, Phalaenopsis, Phyllostachys*, *Physcomitrella*, *Picea, Pyrus, Quercus, Raphanus, Rehmannia, Rubus, Sorghum, Sphenostylis*, *Stellaria, Stylosanthes, Triticum, Trifolium*, *Triticum*, *Vaccinium*, *Vigna*, or *Zinnia* or a microorganism belonging to the genus *Agaricus*, *Aspergillus*, *Ustilago*, *Rhodobacter*, or *Rhodotorula. See*, *e.g.*, WO 2006/089898, Optionally, the PAL is an *Arabidopsis thaliana* PAL, *e.g.*, *A. thaliana* PAL2 (SEQ ID NO:16).

In some embodiments, a tyrosine ammonia lyase (TAL) polypeptide can be expressed, overexpressed, or recombinantly expressed in said microorganism. Optionally, said TAL is a TAL (EC 4.3.1.5) from yeast belonging to the genus *Rhodotorula* or a bacterium belonging to the genus *Rhodobacter. See*, *e.g.*, WO 2006/089898. Optionally, the TAL is a *Rhodobacter capsulatus* TAL, *e.g.*, *R. capsulatus* TAL (SEQ ID NO:17).

In some embodiments, a cinnamate 4-hydroxylase (C4H) polypeptide can be expressed, overexpressed, or recombinantly expressed in said microorganism. Optionally, said C4H is a C4H (EC 1.14.13.11) from a plant belonging to the genus of *Arabidopsis*, *Citrus, Phaseolus*, *Pinus*, *Populus, Solanum, Vitis, Zea, Ammi, Avicennia, Camellia, Camptotheca*, *Catharanthus, Glycine, Helianthus, Lotus, Mesembryanthemum, Physcomitrella*, *Ruta*, *Saccharum*, or *Vigna* or from a microorganism belonging to the genus *Aspergillus. See*, *e.g.*, WO 2006/089898. Optionally, the C4H is *Arabidopsis thaliana* C4H (SEQ ID NO:19).

In some embodiments, a 4-coumarate-CoA ligase (4CL) polypeptide can be expressed, overexpressed, or recombinantly expressed in said microorganism. Optionally, said 4CL can be a 4CL (EC 6.2.1.12) from a plant belonging to the genus of *Abies*, *Arabidopsis, Brassica, Citrus, Larix, Phaseolus, Pinus, Populus*, *Solanum, Vitis, Zea, e.g.,Z. mays, Agastache, Amorpha*, *Cathaya, Cedrus*, *Crocus, Festuca, Glycine, Juglans, Keteleeria, Lithospermum*, *Lolium*, *Lotus, Lycopersicon*, *Malus, Medicago, Mesembryanthemum, Nicotiana, Nothotsuga, Oryza*, *Pelargonium, Petroselinum, Physcomitrella, Picea, Prunus, Pseudolarix*, *Pseudotsuga, Rosa*, *Rubus, Ryza, Saccharum, Suaeda*, *Thellungiella*, *Triticum*, or *Tsuga,* a microorganism belonging to the genus *Aspergillus, Neurospora, Yarrowia*, *Mycosphaeretta*, *Mycobacterium, Neisseria, Streptomyces*, or *Rhodobacter*, or a nematode belonging to the genus *Ancylostoma, Caenorhabditis*, *Haemonchus*, *Lumbricus, Meilodogyne, Strongyloidus,* or *Pristionchus. See*, *e.g.,* WO 2006/089898. Optionally, the 4CL is an *Arabidopsis thaliana* 4CL, *e.g.*, *A. thaliana* 4CL2 (SEQ ID NO:20).

In some embodiments, a stilbene synthase (STS) polypeptide can be expressed, overexpressed, or recombinantly expressed in said microorganism. Optionally, said STS is an STS (EC 2.3.1.95) from a plant belonging to the genus of *Arachis*, *Rheum*, *Vitis, Pinus, Piceea, Lilium, Eucalyptus, Parthenocissus*, *Cissus*, *Calochortus, Polygonum, Gnetum*, *Artocarous*, *Nothofagus*, *Phoenix, Festuca, Carex, Veratrum, Bauhinia,* or *Pterolobium. See*, *e.g.*, WO 2006/089898 . Optionally, the STS is *Vitus pseudoreticulata* STS (SEQ ID NO:21).

Optionally, an NADPH:cytochrome P450 reductase (CPR) polypeptide can be expressed, overexpressed, or recombinantly expressed in said microorganism. Optionally, said CPR is a CPR (EC 1.6.2.4) from a plant belonging to genus *Arabidopsis*, *e.g.*, *A. thaliana*, a plant belonging to genus *Citrus*, *e.g.*, *Citrus x sinensis*, or *Citrus x paradisi*, a plant belonging to genus *Phaseolus*, *e.g.*, *P*. *vulgaris*, a plant belonging to genus *Pinus*, *e.g.*, *P*. *taeda*, a plant belonging to genus *Populus*, *e.g.*, *P*. *deltoides*, *R. tremuloides*, *or R. trichocarpa*, a plant belonging to genus *Solanum*, *e.g.*, *S. tuberosum*, a plant belonging to genus *Vitis*, *e.g.*, *Vitis vinifera*, a plant belonging to genus Zea, *e.g.*, *Z. mays*, or other plant genera, *e.g.*, *Ammi*, *Avicennia*, *Camellia*, *Camptotheca*, *Catharanthus*, *Glycine, Helianthus, Lotus, Mesembryanthemum, Physcomitrella*, *Ruta*, *Saccharum*, or *Vigna. See*, *e.g.*, WO 2006/089898. Optionally, the CPR is an *Arabidopsis thaliana* CPR, *e.g.*, *A thaliana* ATR2 (SEQ ID NO:22).

There is disclosed a recombinant host capable of producing resveratrol which expresses a gene encoding a 3-Deoxy-D-arabinoheptulosonate 7-phosphate (DAHP) synthase [EC 2.5.1.54 ] polypeptide. As used herein, a DAHP synthase polypeptide refers to an enzyme capable of catalyzing the conversion of phosphoenolpyruvate and D-erythrose 4-phosphate to DAHP and phosphate, the first step in aromatic amino acid biosynthesis by the shikimate pathway. Optionally, the DAHP synthase is a *S.cenevisiae* DAHP synthase, *e.g.*, *S*. *cerevisiae* ARO4 (SEQ ID NO:1).

Optionally, a recombinant host capable of producing resveratrol overexpresses a gene encoding a DAHP synthase polypeptide. The overexpressed DAHP synthase gene encodes an ARO4 (SEQ ID NO:1) polypeptide. The recombinant host capable of producing resveratrol overexpresses a DAHP synthase polypeptide that is not feedback-inhibited by aromatic amino acids. For example, an ARO4 K229 mutant polypeptide is not feedback-inhibited by phenylalanine, tyrosine, and/or tryptophan. The ARO4 mutant polypeptide is a K229L mutant (SEQ ID NO:2). A recombinant host overexpressing a gene encoding an ARO4 or ARO4 K229L polypeptide produces resveratrol in greater quantities than a recombinant host not overexpressing an ARO4 or an ARO4 K229L polypeptide. Optionally, the ARO4 mutant polypeptide is a K229P mutant (SEQ ID NO:23). Optionally, a recombinant host overexpressing a gene encoding an ARO4 K229P polypeptide produces resveratrol in greater quantities than a recombinant host not overexpressing an ARO4 K229P polypeptide.

Optionally, a recombinant host capable of producing resveratrol does not express an *S*. *cerevisiae* ARO3 polypeptide (SEQ ID NO:3). Optionally, wherein resveratrol is produced in *S*. *cerevisiae*, the endogenous *S*. *cerevisiae* ARO3 polypeptide is deleted (knocked out). A non-limiting example of a method for knocking out ARO3 is through homologous recombination. *See, e.g.,* Hegemann & Heick, Methods Mol Biol. 765:189-206 (2011).

A recombinant host capable of producing resveratrol further expresses a gene encoding a chorismate mutase [EC 5.4.99.5] polypeptide. As used herein, a chorismate mutase polypeptide refers to an enzyme capable of catalyzing the conversion of chorismate to prephenate, a step in the synthesis of phenylalanine and tyrosine. In some embodiments, the chorismate mutase is an *S*. *cerevisiae* chorismate mutase, *e.g.*, *S. cerevisiae* ARO7 (SEQ ID NO:4).

Optionally, a recombinant host capable of producing resveratrol overexpresses a gene encoding a chorismate mutase polypeptide. Optionally, the overexpressed chorismate mutase gene encodes an ARO7 (SEQ ID NO:4) polypeptide. In some embodiments, the recombinant host capable of producing resveratrol overexpresses a chorismate mutase polypeptide that is not feedback-inhibited by phenylalanine or tyrosine. For example, in some aspects, an ARO7 T226 mutant polypeptide is not feedback-inhibited. In some embodiments, the ARO7 mutant polypeptide is a T226I (SEQ ID NO:5) or T226N polypeptide (SEQ ID NO:15). In some aspects, a recombinant host overexpressing a gene encoding an ARO7, ARO7 T226I, or ARO7 T226N polypeptide produces resveratrol in greater quantities than a recombinant host not overexpressing an ARO7, ARO7 T226I, or ARO7 T226N polypeptide.

in some embodiments, a recombinant host capable of producing resveratrol further expresses a gene encoding an acetyl-CoA carboxylase [EC 6.4.1.2] or [EC 6.3.4.14] polypeptide. As used herein, an acetyl-CoA carboxylase polypeptide refers to a biotin-dependent enzyme capable of catalyzing the carboxylation of acetyl-CoA to produce malonyl-CoA. in some embodiments, the chorismate mutase is an *S*. *cerevisiae* acetyl-CoA carboxylase, *e.g.*, *S. cerevisiae* ACC1 (SEQ ID NO:6).

in some embodiments, a recombinant host capable of producing resveratrol overexpresses a gene encoding an acetyl-CoA carboxylase polypeptide. In some embodiments, the overexpressed chorismate mutase gene encodes an ACC1 (SEQ ID NO:6) polypeptide. Optionally, the recombinant host capable of producing resveratrol overexpresses an acetyl-CoA carboxylase with de-regulated activity. For example, in some aspects, one or more of the phosphorylation sites of ACC1 are mutated by replacing a phosphorylable Serine residue with a non-phosphorylable amino acid. Non-limiting examples of de-regulated ACC1 polypeptides are mutated at Ser659 and/or Ser1157. ACC1 polypeptides can comprise a Ser659A or Ser659V mutation and/or a Ser1157A or Ser1157V mutation. See SEQ ID NOs:7-14. ACC1 polypeptides can comprise a Ser659P mutation and/or a Ser1157P mutation. See SEQ ID NOs:23-25. in some embodiments, a recombinant host overexpressing a gene encoding a de-regulated ACC1 polypeptide produces resveratrol in greater quantities than a recombinant host not overexpressing a de-regulated ACC1 polypeptide.

Resveratrol produced according to the methods disclosed herein can be *cis-*resveratrol or trans-resveratrol, wherein the trans-resveratrol is a predominant species. Resveratrol and resveratrol derivatives formed and/or recovered according to the invention can be analyzed by techniques generally available to one skilled in the art, for example, but not limited to, thin layer chromatography (TLC), high-performance liquid chromatography (HPLC), ultraviolet-visible spectroscopy/spectrophotometry (UV-Vis), liquid chromatography-mass spectrometry (LC-MS), and nuclear magnetic resonance (NMR).

Resveratrol production may be able to be increased by transforming a yeast strain with a plasmid containing a mutated version of the *S*. *cerevisiae* gene *ARO4.* This plasmid allows the integration of the mutated gene in a position of the yeast genome (such as, for example but not limited to, an intergenic region), which is under the control of a strong promoter (*i.e.* a promoter that expresses the gene above native levels, such as, for example but not limited to, pTEF1) causing overexpression of the gene. For example, the *ARO4* gene may contain mutations causing a change in the amino acid 229 from lysine to leucine. Transformants are optionally grown for 72 hours in Delft medium at 30°C and shaken at 400 rpm. Optionally, the extraction of compounds of interest is performed by mixing ethanol with a culture sample to a final concentration of 50%, followed by centrifugation for 5 min at 3222 xg. In some options, the supernatant is analyzed by HPLC detecting, among others, resveratrol, phloretic acid and coumaric acid. As shown in Figure 5, overexpression of this mutated version of *ARO4* increases resveratrol production from approximately 200 mg/L in the control strain transformed with an empty plasmid to approximately 325 mg/L. Furthermore, as shown in Figure 5, overexpression of *ARO4* also increases production of secondary metabolites in the pathway (*i.e*. the pathway intermediate coumaric acid and the side product phloretic acid are represented as secondary metabolites) as compared to the control strain transformed with empty plasmid.

Resveratrol production may be able to be increased by transforming a yeast strain with a plasmid containing mutated versions of the *S*. *cerevisiae* genes *ARO4* and *ARO7.* The *ARO4* gene may contains mutations causing a change in the amino acid 229 from lysine to leucine. The AR07 gene may contain mutations causing a change in the amino acid 226 from threonine to isoleucine. Optionally, the mutated ARO4 and ARO7 genes on the plasmid are inserted in a position of the yeast genome (such as, for example but not limited to, an intergenic region) and are under the control of strong promoters (*i.e.* a promoter that expresses the gene above native levels, such as, for example but not limited to, pTEF1 or pPGK1) causing overexpression of the genes. Transformants may be grown for 72 hours in Delft medium at 30°C and shaken at 400 rpm. The extraction of compounds of interest may be performed by mixing ethanol with a culture sample to a final concentration of 50%, followed by centrifugation for 5 min at 3222 × g. The supernatant may be analyzed by HPLC detecting, among others, resveratrol, phloretic acid and coumaric acid. As shown in Figure 5, overexpression of mutated *ARO4* and *ARO7* genes (ARO4*-ARO7*) increases production of resveratrol from approximately 200 mg/L in the control strain transformed with an empty plasmid to approximately 375 mg/L. Overexpression of mutated ARO4 and ARO7 genes may also increase production of secondary metabolites in the (*i.e.* the pathway intermediate coumaric acid and the side product phloretic acid are represented as secondary metabolites) as compared to the control strain transformed with empty plasmid.

Resveratrol production can be increased by transforming a yeast strain with a plasmid containing mutated versions of the S. *cerevisiae* gene *ARO4.* This plasmid may allow the integration of the mutated gene at the *ARO3* gene causing its disruption. The mutated *ARO4* gene may also be under the control of a strong promoter (*i*.*e*. a promoter that expresses the gene above native levels, such as, for example but not limited to, pTEF1) causing overexpression of the gene. The *ARO4* gene may contain mutations causing a change in the amino acid 229 from lysine to leucine. The same mutated *ARO4* gene may also be inserted in a different position of the yeast genome not affecting the *ARO3* gene, as a control to study the effect of the *ARO3* deletion. The transformants may be grown for 72 hours in Delft medium at 30°C and shaken at 400 rpm. Extraction of the compounds of interest may be performed by mixing ethanol with a culture sample to a final concentration of 50%, followed by centrifugation for 5 min at 3222 × g. As shown in Figure 5, the supernatant is analyzed by HPLC detecting, among others, resveratrol, phloretic acid and coumaric acid. Overexpression of the mutated version of ARO4 combined with the deletion of *ARO3* (aro3::ARO4*) may increase the production of resveratrol from approximately 200 mg/L in the control strain transformed with an empty plasmid to approximately 275 mg/L. Furthermore, as shown in Figure 5, the production of secondary metabolites in the pathway (*i.e*. the pathway intermediate coumaric acid and the side product phloretic acid are represented as secondary metabolites) are also increased for the strain overexpressing the mutated version of *ARO4* combined with the *ARO3* deletion (aro3::ARO4*) over the control strain transformed with an empty plasmid.

Resveratrol production can be increased by transforming a yeast strain with a plasmid containing mutated versions of the *S*. *cerevisiae* genes *ARO4* and *ARO7*. This plasmid may allow the integration of the mutated genes at the *ARO3* gene causing its disruption. The mutated *ARO4* and *ARO7* genes may be under the control of strong promoters (*i*.*e*. a promoter that expresses the gene above native levels, such as, for example but not limited to, pTEF1 or pPGK1) causing overexpression of the genes. The *ARO4* gene may contain mutations causing a change in the amino acid 229 from lysine to leucine. The *AR07* gene may contain mutations causing a change in the amino acid 226 from threonine to isoleucine. The same mutated *ARO4* and *ARO*7 genes may also be inserted in a different position of the yeast genome as a control to study the effect of the *ARO3* deletion. Transformants may be grown for 72 hours in Delft medium at 30°C and shaken at 400 rpm. Extraction of compounds of interest may be performed by mixing ethanol with a culture sample to a final concentration of 50%, followed by centrifugation for 5 min at 3222 xg. The supernatant may be analyzed by HPLC detecting, among others, resveratrol, phloretic acid and coumaric acid. As shown in Figure 5, overexpression of the mutated versions of *ARO4* and *ARO7* combined with the deletion of *ARO3* (aro3::ARO4*-ARO7*) increases production of resveratrol, doubling production from the control strain from approximately 200 mg/L to approximately 400 mg/L, and resulting in increased resveratrol production over the mutated *ARO4* and *ARO7* genes without the *ARO3* deletion, from approximately 375 mg/L to approximately 400 mg/L. Also, as shown in Figure 5, overexpression of the mutated *ARO4* and *ARO7* genes combined with deletion of *ARO3* (aro3::ARO4*-ARO7*) can result in increased production of secondary metabolites in the pathway (*i.e*. the pathway intermediate coumaric acid and the side product phloretic acid are represented as secondary metabolites) over either the control strain transformed with empty plasmid or overexpression of mutated *ARO4* and *ARO7* genes without deletion of *ARO3* (ARO4*-ARO7*).

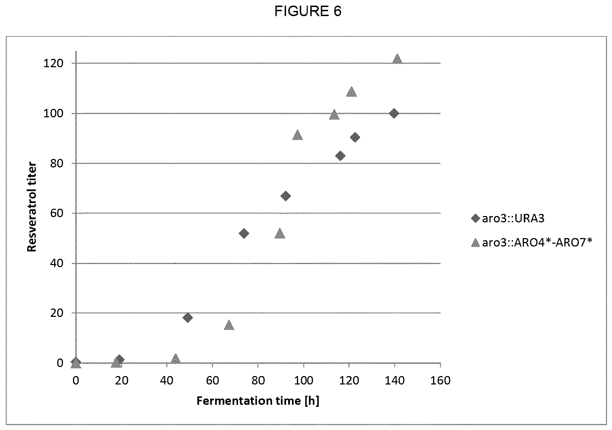

Resveratrol production can be increased by transforming a yeast strain with a plasmid containing mutated versions of the *S*. *cerevisiae* genes *ARO4* and *ARO7*. This plasmid may allow the integration of the mutated genes at the *ARO3* gene causing its disruption. The mutated *ARO4* and *ARO7* genes may be under the control of strong promoters (*i.e*. a promoter that expresses the gene above native levels, such as, for example but not limited to, pTEF1 or pPGK1) causing overexpression of the genes. The ARO4 gene may contain mutations causing a change in the amino acid 229 from lysine to leucine. The ARO7 gene may contain mutations causing a change in the amino acid 226 from threonine to isoleucine. The resulting strain may be later transformed with a DNA fragment comprising the URA3 selection marker and flanking regions, facilitating deletion of the introduced ARO4* and ARO7*. Cultivation of the two strains may be performed in bioreactors via fed-batch culture. Extraction of compounds of interest may be performed by mixing ethanol with a culture sample to a final concentration of 50%, followed by centrifugation for 5 min at 3222 xg. The supernatant may be analyzed by HPLC detecting, among others, resveratrol, phloretic acid and coumaric acid. As shown in Figure 6, the results show that the strain overexpressing *ARO4* and *ARO7* combined with deletion of *ARO3* (aro3::ARO4*-ARO7*)may result in increased resveratrol titers by 20% over the strain with deletion of *ARO3, ARO4** and *ARO7** (aro3::URA3). Furthermore, as shown in Figure 7, the strain overexpressing the mutated *ARO4* and *ARO7* genes combined with deletion of ARO3 (aro3::ARO4*-ARO7*)may result in approximately a 100% increase in total titer of resveratrol and secondary metabolites in the pathway (*i*.*e*. pathway intermediate coumaric acid and the side product phloretic acid are represented as secondary metabolites) over the strain with deletion of *ARO3, ARO4** and *ARO7** (aro3::URA3).

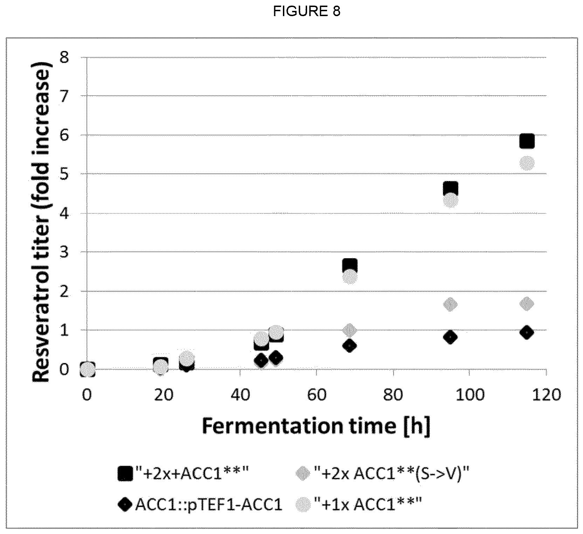

Resveratrol production can be increased by transforming a yeast strain with a plasmid containing a mutated *ACC1* or an additional resveratrol synthase under the control of a constitutive promoter (pTEF1; SEQ ID NO:26). The mutated ACC1 may carry mutations conferring the residue changes S659A and S1157A; this mutant (SEQ ID NO:11) will be referred to as ACC1**. Another resveratrol producing yeast strain, a mutated *ACC1* may be introduced in the same locus carrying mutations conferring the residue changes S659V and S1157V, this mutant (SEQ ID NO:14) will be referred to as ACC1**(S->V). In some cases, the plasmid provided for integration of a single copy into the yeast genome, thereby only integrating the promoters, terminators, and genes included in the cassette in an intergenic region, without integration of the plasmid backbone itself. Another round of integrations may be carried out to introduce a second copy of the genes mentioned above. As a control to the mutated *ACC1,* the endogenous *ACC1* gene may be overexpressed by promoter replacement in a resveratrol-producing yeast strain without any additional integrated copies of mutated *ACC1* (ACC1::pTEF1-ACC1). The promoter replacement may be done by introduction of a pTEF1 promoter sequence in between the 5' UTR of *ACC1* and its start codon. Cultivation of the transformant strains may be performed in bioreactors via fed-batch culture. Exraction of compounds may be carried out by mixing ethanol with a culture sample to a final concentration of 50%, and centrifugation for 5 minutes at 3222 x g. As shown in Figure 8, the results obtained show: 1) that introduction of a mutated *ACC1* ** resulted in elevated levels of Resveratrol final titer; 2) that both *ACC1* mutant versions (*ACC1*** and *ACC1*** (S->V)) resulted in an increased Resveratrol final titer; and 3) that overexpression of a wild-type *ACC1* alone did not demonstrate this improvement.

### Resveratrol Modifications

A stilbene or a modified stilbene may be produced by bioconversion or in a cell. Optionally, the stilbene is resveratrol or a resveratrol derivative. As used herein, the terms "modified resveratrol," "resveratrol derivative," and "resveratrol analog" can be used interchangeably to refer to a compound that can be derived from resveratrol or a compound with a similar structure to resveratrol. As used herein, the terms "resveratrol derivative" or "resveratrol analog" can be used interchangeably to refer to resveratrol-like molecules. Resveratrol derivatives may be salts and esters of resveratrol or analogs or derivatives thereof.

Additional non-limiting examples of resveratrol analogs or derivatives thereof include hydroxylated resveratrol analogs or derivatives such as hydroxystilbene, dihydroxystilbene, 3,5-dihydroxypterostilbene, tetrahydroxystilbene, pentahydroxystilbene, or hexahydroxystilbene, fluorinated stilbenes, bridged stilbenes, digalloylresveratrol (ester of gallic acid and resveratrol), or resveratrol triacetate.

Advantageously, the resveratrol preparations of the invention have a purity defined herein as a lack or absence of chemical, biochemical or biologic contaminants present in resveratrol preparations prepared from natural sources. Potentially resveratrol preparations provided by the invention do not contain emodin, a plant contaminant present in resveratrol extracted from knotweed having laxative properties not desired for many applications of resveratrol.

A composition containing resveratrol or an analog or derivative thereof can be formulated into a composition and administered to a subject by any suitable route of administration, including oral or parenteral routes of administration. Specific administration modalities include subcutaneous, intravenous, intramuscular, intraperitoneal, transdermal, intrathecal, oral, rectal, buccal, topical, nasal, ophthalmic, intra articular, intra-arterial, sub arachnoid, bronchial, lymphatic, vaginal, and intra uterine administration. Optionally, the composition can be in the form of a capsule, liquid (*e.g*., a beverage), tablet, pill, gel, pellet, foodstuff, dry or wet animal feed, or formulated for prolonged release. In some embodiments, a resveratrol composition can be a solution. Any of the compositions described herein can be included in a container, pack, or dispenser together with instructions for administration. Optionally, the composition is packaged as a single use vial.

### Functional Homologs

Functional homologs of the polypeptides described above are also suitable for use in producing resveratrol derivatives. A functional homolog is a polypeptide that has sequence similarity to a reference polypeptide, and that carries out one or more of the biochemical or physiological function(s) of the reference polypeptide. A functional homolog and the reference polypeptide can be natural occurring polypeptides, and the sequence similarity can be due to convergent or divergent evolutionary events. As such, functional homologs are sometimes designated in the literature as homologs, or orthologs, or paralogs. Variants of a naturally occurring functional homolog, such as polypeptides encoded by mutants of a wild type coding sequence, can themselves be functional homologs. Functional homologs can also be created via site-directed mutagenesis of the coding sequence for a polypeptide, or by combining domains from the coding sequences for different naturally-occurring polypeptides ("domain swapping"). Techniques for modifying genes encoding functional polypeptides described herein are known and include, *inter alia*, directed evolution techniques, site-directed mutagenesis techniques and random mutagenesis techniques, and can be useful to increase specific activity of a polypeptide, alter substrate specificity, alter expression levels, alter subcellular location, or modify polypeptide-polypeptide interactions in a desired manner. Such modified polypeptides are considered functional homologs. The term "functional homolog" is sometimes applied to the nucleic acid that encodes a functionally homologous polypeptide.

Functional homologs can be identified by analysis of nucleotide and polypeptide sequence alignments. For example, performing a query on a database of nucleotide or polypeptide sequences can identify homologs of polypeptides described herein. Sequence analysis can involve BLAST, Reciprocal BLAST, or PSI-BLAST analysis of nonredundant databases using the amino acid sequence of interest as the reference sequence. Amino acid sequence is, in some instances, deduced from the nucleotide sequence. Those polypeptides in the database that have greater than 40% sequence identity are candidates for further evaluation for suitability as polypeptide useful in the synthesis of resveratrol and resveratrol derivatives. Amino acid sequence similarity allows for conservative amino acid substitutions, such as substitution of one hydrophobic residue for another or substitution of one polar residue for another. When desired, manual inspection of such candidates can be carried out in order to narrow the number of candidates to be further evaluated. Manual inspection can be performed by selecting those candidates that appear to have conserved functional domains.

Conserved regions can be identified by locating a region within the primary amino acid sequence of a polypeptide described herein that is a repeated sequence, forms some secondary structure (*e*.*g*., helices and beta sheets), establishes positively or negatively charged domains, or represents a protein motif or domain. *See, e.g.,* the Pfam web site describing consensus sequences for a variety of protein motifs and domains on the World Wide Web at sanger.ac.uk/Software/Pfam/ and pfam.janelia.org/. The information included at the Pfam database is described in Sonnhammer et al., 1998, Nucl. Acids Res., 26:320-322; Sonnhammer et al., 1997, Proteins, 28:405-420; and Bateman et al., 1999, Nucl. Acids Res., 27:260-262. Conserved regions also can be determined by aligning sequences of the same or related polypeptides from closely related species. Closely related species preferably are from the same family. It may be that alignment of sequences from two different species can be adequate.

Typically, polypeptides that exhibit at least about 40% amino acid sequence identity are useful to identify conserved regions. Conserved regions of related polypeptides exhibit at least 45% amino acid sequence identity (*e.g*., at least 50%, at least 60%, at least 70%, at least 80%, or at least 90% amino acid sequence identity). Optionally, a conserved region exhibits at least 92%, 94%, 96%, 98%, or 99% amino acid sequence identity.

A percent identity for any candidate nucleic acid or polypeptide relative to a reference nucleic acid or polypeptide can be determined as follows. A reference sequence (*e.g*., a nucleic acid sequence or an amino acid sequence) is aligned to one or more candidate sequences using the computer program ClustalW (version 1.83, default parameters), which allows alignments of nucleic acid or polypeptide sequences to be carried out across their entire length (global alignment). See Chenna et al., 2003, Nucleic Acids Res., 31(13):3497-500. ClustalW can be run, for example, at the Baylor College of Medicine Search Launcher site on the World Wide Web (searchlauncher.bcm.tmc.edu/multi-align/multi-align.html) and at the European Bioinformatics Institute site on the World Wide Web (ebi.ac.uk/clustalw).

To determine percent identity of a candidate nucleic acid or amino acid sequence to a reference sequence, the sequences are aligned using ClustalW, the number of identical matches in the alignment is divided by the length of the reference sequence, and the result is multiplied by 100.

It will be appreciated that polypeptides described herein can include additional amino acids that are not involved in enzymatic activities carried out by the enzyme, and thus such a polypeptide can be longer than would otherwise be the case. For example, a polypeptide can include a purification tag (*e.g*., HIS tag or GST tag), a chloroplast transit peptide, a mitochondrial transit peptide, an amyloplast peptide, signal peptide, or a secretion tag added to the amino or carboxy terminus. In some embodiments, a polypeptide includes an amino acid sequence that functions as a reporter, *e.g.,* a green fluorescent protein or yellow fluorescent protein.

### Recombinant Microorganisms

A number of prokaryotes and eukaryotes are suitable for use in constructing the recombinant microorganisms described herein, *e*.*g*., bacteria, yeast and fungi. A species and strain selected for use as a strain for production of resveratrol or resveratrol derivatives first analyzed to determine which production genes are endogenous to the strain and which genes are not present (*e*.*g*., resveratrol production genes). Genes for which an endogenous counterpart is not present in the strain are assembled in one or more recombinant constructs, which are then transformed into the strain in order to supply the missing function(s).

In the present context the terms "microorganism" and "microorganism host" and "recombinant host" can be used interchangeably to refer to microscopic organisms, including bacteria or microscopic fungi, including yeast. Specifically, the microorganism can be a eukaryotic cell or immortalized cell.

Exemplary prokaryotic and eukaryotic species are described in more detail below. However, it will be appreciated that other species can be suitable. For example, suitable species can be in a genus including *Agaricus*, *Aspergillus*, *Bacillus*, *Candida*, *Corynebacterium, Escherichia*, *Fusarium*/*Gibberella, Kluyveromyces*, *Laetiporus*, *Lentinus*, *Phaffia*, *Phanerochaete, Pichia, Physcomitrella*, *Rhodoturula*, *Saccharomyces, Schizosaccharomyces*, *Sphaceloma*, *Xanthophyllomyces* and *Yarrowia.* Exemplary species from such genera include *Lentinus tigrinus*, *Laetiporus sulphureus*, *Phanerochaete chrysosporium*, *Pichia pastoris*, *Physcomitrella patens*, *Rhodoturula glutinis* 32, *Rhodoturula mucilaginosa*, *Phaffia rhodozyma* UBV-AX, *Xanthophyllomyces dendrorhous*, *Fusarium fujikuroi*/*Gibberella fujikuroi*, *Candida utilis* and *Yarrowia lipolytica.* In some embodiments, a microorganism can be an Ascomycete such as *Gibberella fujikuroi*, *Kluyveromyces lactis*, *Schizosaccharomyces pombe*, *Aspergillus niger*, or *Saccharomyces cerevisiae.* In some embodiments, a microorganism can be a prokaryote such as *Escherichia coli*, *Rhodobacter sphaeroides*, or *Rhodobacter capsulatus.* It will be appreciated that certain microorganisms can be used to screen and test genes of interest in a high throughput manner, while other microorganisms with desired productivity or growth characteristics can be used for large-scale production of resveratrol or resveratrol derivatives or analogs.

Suitable microorganisms include, but are not limited to, *S*. *cerevisiae*, *A. niger*, *A. oryzae*, *E. coli*, *L. lactis* and *B. subtilis.* The constructed and genetically engineered microorganisms provided by the invention can be cultivated using conventional fermentation processes, including, *inter alia*, chemostat, batch, fed-batch cultivations, continuous perfusion fermentation, and continuous perfusion cell culture.

Exemplary embodiments comprising bacterial cells include, but are not limited to, cells of species, belonging to the genus *Bacillus*, the genus *Escherichia,* the genus *Lactobacillus,* the genus *Lactobacillus,* the genus *Corynebacterium*, the genus *Acetobacler*, the genus *Acinetobacler*, or the genus *Pseudomonas.*

The microorganism can be a fungus, and more specifically, a filamentous fungus belonging to the genus of *Aspergillus*, *e.g.*, *A. niger*, *A. awamori*, *A. oryzae*, or *A. nidulans*, a yeast belonging to the genus of *Saccharomyces*, *e.g., S. cerevisiae*, *S. kluyveri*, *S. bayanus*, *S. exiguus*, *S. sevazzi*, or *S*. *uvarum*, a yeast belonging to the genus *Kluyveromyces*, *e.g.*, *K. laclis*, *K. marxianus var. marxianus*, or *K. thermololerans*, a yeast belonging to the genus Candida, *e.g.*, *C. ulilis*, *C. Iropicalis*, *C. albicans*, *C. lipolylica*, or *C*. *versalilis*, a yeast belonging to the genus *Pichia, e.g.*, *R. slipidis*, *R. pasloris*, or *P*. *sorbilophila*, or other yeast genera, *e.g.*, *Cryptococcus*, *Debaromyces*, *Hansenula*, *Pichia*, *Yarrowia*, *Zygosaccharomyces*, or *Schizosaccharomyces.* Concerning other microorganisms a non-exhaustive list of suitable filamentous fungi is supplied: a species belonging to the genus *Penicillium*, *Rhizopus*, *Fusarium*, *Fusidium*, *Gibberella*, *Mucor*, *Morlierella*, and *Trichoderma.*

### Saccharomyces cerevisiae

*Saccharomyces cerevisiae* is a widely used chassis organism in synthetic biology, and can be used as the recombinant microorganism platform. There are libraries of mutants, plasmids, detailed computer models of metabolism and other information available for *S*. *cerevisiae,* allowing for rational design of various modules to enhance product yield. Methods are known for making recombinant microorganisms.

The genes described herein can be expressed in yeast using any of a number of known promoters. Strains that overproduce phenylpropanoids are known and can be used as acceptor molecules in the production of resveratrol or resveratrol derivatives.

### Aspergillus spp.

*Aspergillus* species such as *A*. *oryzae*, *A. niger* and *A*. *sojae* are widely used microorganisms in food production, and can also be used as the recombinant microorganism platform. Nucleotide sequences are available for genomes of *A*. *nidulans*, *A. fumigatus*, *A. oryzae*, *A. clavatus*, *A. flavus*, *A. niger*, and *A*. *terreus*, allowing rational design and modification of endogenous pathways to enhance flux and increase product yield. Metabolic models have been developed for *Aspergillus*, as well as transcriptomic studies and proteomics studies. *A. niger* is cultured for the industrial production of a number of food ingredients such as citric acid and gluconic acid, and thus species such as *A. niger* are generally suitable for the production of resveratrol and resveratrol derivatives.

### Escherichia coli

*Escherichia coli,* another widely used platform organism in synthetic biology, can also be used as the recombinant microorganism platform. Similar to *Saccharomyces*, there are libraries of mutants, plasmids, detailed computer models of metabolism and other information available for *E*. *coli*, allowing for rational design of various modules to enhance product yield. Methods similar to those described above for *Saccharomyces* can be used to make recombinant *E*. *coli* microorganisms.

### Agaricus, Gibberella, and Phanerochaete spp.

*Agaricus, Gibberella,* and *Phanerochaete* spp. can be useful because they are known to produce large amounts of gibberellin in culture. Thus, the precursors of terpenes used as acceptor molecules in the production of resveratrol or resveratrol derivatives are already produced by endogenous genes. Thus, modules containing recombinant genes for biosynthesis of terpenes can be introduced into species from such genera without the necessity of introducing other compounds or pathway genes.

### Rhodobacter spp.

*Rhodobacter* can be used as the recombinant microorganism platform. Similar to *E*. *coli,* there are libraries of mutants available as well as suitable plasmid vectors, allowing for rational design of various modules to enhance product yield. Isoprenoid pathways have been engineered in membraneous bacterial species of *Rhodobacter* for increased production of carotenoid and CoQ10. See, U.S. Patent Publication Nos. 20050003474 and 20040078846. Methods similar to those described above for *E*. *coli* can be used to make recombinant *Rhodobacter* microorganisms.

### Phvscomitrella spp.

*Physcomitrella* mosses, when grown in suspension culture, have characteristics similar to yeast or other fungal cultures. This genera is becoming an important type of cell for production of plant secondary metabolites, which can be difficult to produce in other types of cells.

As will be apparent to one skilled in the art, the particulars of the selection process for specific resveratrol derivatives depend on the identities of the selectable markers. Selection in all cases promotes or permits proliferation of cells comprising the marker while inhibiting or preventing proliferation of cells lacking the marker. If a selectable marker is an antibiotic resistance gene, the transfected host cell population can be cultured in the presence of an antibiotic to which resistance is conferred by the selectable marker. If a selectable marker is a gene that complements an auxotrophy of the host cells, the transfected host cell population can be cultivated in the absence of the compound for which the host cells are auxotrophic.

After selection, recombinant host cells can be cloned according to any appropriate method known in the art. For example, recombinant microbial host cells can be plated on solid media under selection conditions, after which single clones can be selected for further selection, characterization, or use. This process can be repeated one or more times to enhance stability of the expression construct within the host cell. To produce resveratrol derivatives, recombinant host cells comprising one or more expression vectors can be cultured to expand cell numbers in any appropriate culturing apparatus known in the art, such as a shaken culture flask or a fermenter.

Culture media used for various recombinant host cells are well known in the art. Culture media used to culture recombinant bacterial cells will depend on the identity of the bacteria. Culture media used to culture recombinant yeast cells will depend on the identity of the yeast. Culture media generally comprise inorganic salts and compounds, amino acids, carbohydrates, vitamins and other compounds that are either necessary for the growth of the host cells or improve health or growth or both of the host cells.

As used herein, the term "fed-batch culture" or "semi-batch culture" are used interchangeably to refer to as an operational technique in biotechnological processes where one or more nutrients (substrates) are fed (supplied) to the bioreactor during cultivation and in which the product(s) remain in the bioreactor until the end of the run. In some embodiments, all the nutrients are fed into the bioreactor.

The invention will be further described in the following examples, which do not limit the scope of the invention described in the claims.

### EXAMPLES

The Examples that follow are illustrative of specific embodiments of the invention and various uses thereof. They are set forth for explanatory purposes only, and are not to be taken as limiting the invention. Any examples falling outside of the scope of protection afforded by the claims are provided for reference only.

### Example 1: Resveratrol production in a resveratrol-producing strain overexpressing mutated ARO4

A resveratrol-producing yeast strain comprising wild-type *ARO3, ARO4* and *ARO7* was transformed with a plasmid containing a mutated version of the *S*. *cerevisiae* gene *ARO4.* This plasmid allowed integration of the mutated gene into the yeast genome, under the control of a strong promoter (pTEF1) causing overexpression of the gene. The integration occurred either in an intergenic region or at the *ARO3* locus, deleting that ORF at the same time. Besides the inserted gene(s), no traces of the plasmids are inserted in the genome of the host strain. This version of the *ARO4* gene contained mutations causing a change in the amino acid 229 from lysine to leucine. Transformants were grown for 72 hours in Delft medium at 30°C and shaken at 400 rpm. Extraction of compounds of interest was performed by mixing ethanol with a culture sample to a final concentration of 50%, followed by centrifugation for 5 min at 3222 × g. The supernatant was analyzed by HPLC detecting, among others, resveratrol, phloretic acid and coumaric acid. The results obtained show that overexpression of the mutated version of *ARO4* increased resveratrol production from approximately 200 mg/L in the control strain transformed with an empty plasmid to approximately 325 mg/L. (Figure 5). Overexpression of the mutated *ARO4* gene also increased production of secondary metabolites in the pathway (*i*.*e*. the pathway intermediate coumaric acid and the side product phloretic acid are represented as secondary metabolites) as compared to the control strain transformed with empty plasmid. (Figure 5).

### Example 2: Resveratrol production in a resveratrol-producing strain overexpressing mutated ARO4 and mutated ARO7

A resveratrol-producing yeast strain comprising wild-type *ARO3, ARO4* and *ARO7* was transformed with a plasmid containing mutated versions of the *S*. *cerevisiae* genes *ARO4* and *ARO7*. This version of the *ARO4* gene contained mutations causing a change in the amino acid 229 from lysine to leucine. This version of the *ARO7* gene contained mutations causing a change in the amino acid 226 from threonine to isoleucine. The mutated *ARO4* and *ARO7* genes on the plasmid were inserted in an intergenic region of the yeast genome and were under the control of strong promoters (pTEF1 and pPGK1, respectively) causing overexpression of the genes. Transformants were grown for 72 hours in Delft medium at 30°C and shaken at 400 rpm. Extraction of compounds of interest was performed by mixing ethanol with a culture sample to a final concentration of 50%, followed by centrifugation for 5 min at 3222 × g. The supernatant was analyzed by HPLC detecting, among others, resveratrol, phloretic acid and coumaric acid. The results obtained show that overexpression of mutated *ARO4* and *ARO7* genes (ARO4*-ARO7*) increased the production of resveratrol from approximately 200 mg/L in the control strain transformed with an empty plasmid to approximately 375 mg/L. (Figure 5). Overexpression of mutated *ARO4* and *ARO7* also increased production of secondary metabolites in the pathway (*i.e.* the pathway intermediate coumaric acid and the side product phloretic acid are represented as secondary metabolites) as compared to the control strain transformed with empty plasmid. (Figure 5).

### Example 3: Resveratrol production in a resveratrol-producing ARO3 knockout strain overexpressing mutated ARO4

A resveratrol-producing yeast strain comprising wild-type *ARO3, ARO4* and *ARO7* was transformed with a plasmid containing a mutated version of the S. *cerevisiae* gene *ARO4.* This plasmid allowed integration of the mutated gene at the *ARO3* locus, causing disruption of *ARO3.* The mutated *ARO4* gene was also under the control of a strong promoter (pTEF1) causing overexpression of the gene. This version of the *ARO4* gene contained mutations causing a change in the amino acid 229 from lysine to leucine. As detailed in Example 1, the same mutated *ARO4* gene was also inserted in an intergenic region of the yeast genome, which did not affect the *ARO3* gene, as a control to study the effect of the *ARO3* deletion. Transformants were grown for 72 hours in Delft medium at 30°C and shaken at 400 rpm. Extraction of compounds of interest was performed by mixing ethanol with a culture sample to a final concentration of 50%, followed by centrifugation for 5 min at 3222 × g. The supernatant was analyzed by HPLC detecting, among others, resveratrol, phloretic acid and coumaric acid. The results showed that overexpression of the mutated version of *ARO4* combined with deletion of *ARO3* (aro3::ARO4*) increased production of resveratrol from approximately 200 mg/L in the control strain transformed with an empty plasmid to approximately 275 mg/L. (Figure 5). However, as detailed in Example 1, overexpression of the mutated version of *ARO4* without the ARO3 deletion (ARO4*) resulted in production of approximately 325 mg/L of resveratrol, approximately 50 mg/L more resveratrol than the mutated *ARO4* gene combined with the deletion of *ARO3*. (Figure 5). Production of secondary metabolites in the pathway (*i*.*e*. pathway intermediate coumaric acid and the side product phloretic acid are represented as secondary metabolites) also increased in the strain overexpressing the mutated version of ARO4 combined with the *ARO3* deletion (aro3::ARO4*) over the control strain transformed with an empty plasmid. (Figure 5).

### Example 4: Resveratrol production in a resveratrol-producing ARO3 knockout strain overexpressing mutated ARO4 and mutated ARO7

A resveratrol-producing yeast strain comprising wild-type *ARO3, ARO4* and *ARO7* was transformed with a plasmid containing mutated versions of the S. *cerevisiae* genes *ARO4* and *ARO7*. This plasmid allowed integration of the mutated genes at the *ARO3* locus causing its disruption. The mutated *ARO4* and *ARO7* genes were under the control of strong promoters (pTEF1 and pPGK1, respectively) causing overexpression of the genes. This version of the *ARO4* gene contained mutations causing a change in the amino acid 229 from lysine to leucine. This version of the *ARO7* gene contained mutations causing a change in the amino acid 226 from threonine to isoleucine. The same mutated *ARO4* and *ARO7* genes were also inserted in an intergenic region of the yeast genome as a control to study the effect of the *ARO3* deletion. (*See* Example 2). Transformants were grown for 72 hours in Delft medium at 30°C and shaken at 400 rpm. Extraction of compounds of interest was performed by mixing ethanol with a culture sample to a final concentration of 50%, followed by centrifugation for 5 min at 3222 × g. The supernatant was analyzed by HPLC detecting, among others, resveratrol, phloretic acid and coumaric acid. The results showed that overexpression of the mutated versions of *ARO4* and *ARO7* combined with deletion of *ARO3* (aro3::ARO4*-ARO7*) increased production of resveratrol, doubling production from the control strain from approximately 200 mg/L to approximately 400 mg/L, and resulting in increased resveratrol production over the mutated *ARO4* and *ARO7* genes without the *ARO3* deletion, from approximately 375 mg/L to approximately 400 mg/L. (Figure 5). Also, overexpression of the mutated *ARO4* and *ARO7* genes combined with deletion of *ARO3* (aro3::ARO4*-ARO7*) resulted in increased production of secondary metabolites in the pathway (*i*.*e*. pathway intermediate coumaric acid and the side product phloretic acid are represented as secondary metabolites) over either the control strain transformed with empty plasmid or overexpression of mutated *ARO4* and *ARO7* genes without deletion of *ARO3* (ARO4*-ARO7*). (Figure 5).

### Example 5: Resveratrol production in a resveratrol-producing ARO3 knockout strain overexpressing mutated ARO4 and mutated ARO7

A resveratrol-producing yeast strain comprising wild-type *ARO3, ARO4* and *ARO7* was transformed with a plasmid containing mutated versions of the *S*. *cerevisiae* genes *ARO4* and *ARO7*. This plasmid allowed integration of the mutated genes at the *ARO3* locus causing its disruption. The mutated *ARO4* and *ARO7* genes were under the control of strong promoters (pTEF1 and pPGK1, respectively) causing overexpression of the genes. This version of the *ARO4* gene contained mutations causing a change in the amino acid 229 from lysine to leucine. This version of the *ARO7* gene contained mutations causing a change in the amino acid 226 from threonine to isoleucine. The resulting strain was later transformed with a DNA fragment comprising the URA3 selection marker and flanking regions, facilitating deletion of the introduced ARO4* and ARO7*. Cultivation of the two strains was performed in bioreactors via fed-batch culture. Extraction of compounds of interest was performed by mixing ethanol with a culture sample to a final concentration of 50%, followed by centrifugation for 5 min at 3222 × g. The supernatant was analyzed by HPLC detecting, among others, resveratrol, phloretic acid and coumaric acid. The results show that the strain overexpressing ARO4 and ARO7 combined with deletion of ARO3 (aro3::ARO4*-ARO7*) resulted in increased resveratrol titers by 20% over the strain with deletion of *ARO3, ARO4** and *ARO7** (aro3::URA3). (Figure 6). In addition, the strain overexpressing the mutated *ARO4* and *ARO7* genes combined with deletion of *ARO3* (aro3::ARO4*-ARO7*) resulted in approximately a 100% increase in total titer of resveratrol and secondary metabolites in the pathway (*i*.*e*. pathway intermediate coumaric acid and the side product phloretic acid are represented as secondary metabolites) over the strain with deletion of *ARO3, ARO4** and ARO7* (aro3::URA3) (Figure 7).

### Example 6: Resveratrol production in a resveratrol-producing strain overexpressing a mutated ACC1

A resveratrol-producing yeast strain was transformed with a plasmid containing a mutated *ACC1* or an additional resveratrol synthase under the control of a constitutive promoter (pTEF1; SEQ ID NO:26). The mutated *ACC1* carried mutations conferring the residue changes S659A and S1157A; this mutant (SEQ ID NO:11) will be referred to as ACC1**. In another resveratrol-producing yeast strain, a mutated *ACC1* was introduced in the same locus carrying mutations conferring the residue changes S659V and S1157V, this mutant (SEQ ID NO:14) will be referred to as ACC1**(S->V). The plasmid provided for integration of a single copy into the yeast genome, thereby only integrating the promoters, terminators, and genes included in the cassette in an intergenic region, without integration of the plasmid backbone itself. Another round of integrations was carried out to introduce a second copy of the genes mentioned above. As a control to the mutated *ACC1*, the endogenous *ACC1* gene was overexpressed by promoter replacement in a resveratrol-producing yeast strain without any additional integrated copies of mutated *ACC1* (ACC1::pTEF1-ACC1). The promoter replacement was done by introduction of a pTEF1 promoter sequence in between the 5' UTR of *ACC1* and its start codon. Cultivation of the transformant strains was performed in bioreactors via fed-batch culture. Extraction of compounds was carried out by mixing ethanol with a culture sample to a final concentration of 50%, and centrifugation for 5 minutes at 3222 × g. The results obtained show: 1) that introduction of a mutated *ACC1*** resulted in elevated levels of Resveratrol final titer; 2) that both *ACC1* mutant versions (*ACC1*** and *ACC1*** (S->V)) resulted in an increased Resveratrol final titer; and 3) that overexpression of a wild-type *ACC1* alone did not demonstrate this improvement (Figure 8).

Having described the invention in detail and by reference to specific embodiments thereof, it will be apparent that modifications and variations are possible without departing from the scope of the invention defined in the appended claims. More specifically, although some aspects of the present invention are identified herein as particularly advantageous, it is contemplated that the present invention is not necessarily limited to these particular aspects of the invention. Rather, the scope of the invention is defined by the appended claims.

## Claims

1. A recombinant host microorganism cell comprising:
(a) a gene encoding a tyrosine-sensitive 3-Deoxy-D-arabinoheptulosonate 7-phosphate synthase (ARO4) polypeptide having at least 65% identity to the amino acid sequence set forth in SEQ ID NO: 1, wherein the ARO4 polypeptide comprises a mutation that is K229L and is not feedback inhibited;
(b) a gene encoding a chorismate mutase polypeptide;
(c) a gene encoding an acetyl-CoA carboxylase polypeptide; and
(d) disruption of a gene encoding a phenylalanine-inhibited 3-Deoxy-D-arabinoheptulosonate 7-phosphate synthase (ARO3) polypeptide having at least 75% identity to the amino acid sequence set forth in SEQ ID NO: 3, whereby the host does not express ARO3;
wherein at least one of the genes is a recombinant gene,
wherein the host is capable of producing a stilbene.

2. The host of claim 1, wherein the chorismate mutase polypeptide comprises a chorismate mutase (ARO7) polypeptide having at least 50% identity to the amino acid sequence set forth in SEQ ID NO: 4; wherein the chorismate mutase polypeptide comprises an ARO7 polypeptide having a mutation that is T226I or T226N, and wherein the chorismate mutase polypeptide is not feedback inhibited.

3. The host of claim 1 or 2, wherein the acetyl-CoA carboxylase polypeptide comprises an acetyl-CoA carboxylase alpha (ACC1) polypeptide having at least 70% identity to the amino acid sequence set forth in SEQ ID NO: 6, having a mutation at S659 or S1157, wherein the replacement amino acid is non-phosphorylable and the ACC1 polypeptide is not feedback inhibited.

4. The host of claim 3, wherein the acetyl-CoA carboxylase polypeptide comprises an ACC1 polypeptide having at least one mutation that is S659A, S659V, S1157A, or S1157V.

5. The host of any preceding claim, further comprising one or more of:
(a) a gene encoding a L-phenylalanine ammonia lyase (PAL) polypeptide;
(b) a gene encoding a cinnamate-4-hydroxylase (C4H) polypeptide;
(c) a gene encoding a NADPH:cytochrome P450 reductase polypeptide;
(d) a gene encoding a tyrosine ammonia lyase (TAL) polypeptide;
(e) a gene encoding a 4-coumarate-CoA ligase (4CL) polypeptide; or
(f) a gene encoding a stilbene synthase (STS) polypeptide;
wherein at least one of the genes is a recombinant gene.

6. The host of any one of claims 1-5, wherein the stilbene is resveratrol or a resveratrol derivative.

7. The host of claim 6, wherein recombinant host microorganism cell comprises *Escherichia* bacteria cells, *Lactobacillus* bacteria cells, *Lactococcus* bacteria cells, *Cornebacterium* bacteria cells, *Acetobacter* bacteria cells, *Acinetobacter* bacteria cells, or *Pseudomonas* bacterial cells.

8. The host of claim 6, wherein recombinant host microorganism cell is a cell from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous*, or *Candida albicans* species.

9. A method of producing a stilbene, comprising:
(a) growing a recombinant host microorganism in a culture medium, under conditions in which the genes are expressed,
wherein the host comprises:
(i) a gene encoding a tyrosine-sensitive 3-Deoxy-D-arabinoheptulosonate 7-phosphate synthase (ARO4) polypeptide having at least 65% identity to the amino acid sequence set forth in SEQ ID NO: 1, wherein the ARO4 polypeptide comprises a mutation that is K229L and is not feedback inhibited;
(ii) a gene encoding a chorismate mutase polypeptide;
(iii) a gene encoding an acetyl-CoA carboxylase polypeptide; and
(iv) disruption of a gene encoding a phenylalanine-inhibited 3-Deoxy-D-arabinoheptulosonate 7-phosphate synthase (ARO3) polypeptide having at least 75% identity to the amino acid sequence set forth in SEQ ID NO: 3, whereby the host does not express ARO3;
wherein at least one of the genes is a recombinant gene,
and, optionally
(b) recovering the stilbene from the culture media.

10. The method of claim 9, wherein the chorismate mutase polypeptide comprises an ARO7 polypeptide having at least 50% identity to the amino acid sequence set forth in SEQ ID NO: 4, having a mutation that is T226I or T226N, and wherein the chorismate mutase polypeptide is not feedback inhibited.

11. The method of claim 9 or 10, wherein the acetyl-CoA carboxylase polypeptide comprises an ACC1 polypeptide having at least 70% identity to the amino acid sequence set forth in SEQ ID NO:6, having a mutation at S659 or S1157, wherein the replacement amino acid is non-phosphorylable and the acetyl-CoA carboxylase polypeptide is not feedback inhibited.

12. The method of claim 11, wherein the acetyl-CoA carboxylase polypeptide comprises an ACC1 polypeptide having at least one mutation that is S659A, S659V, S1157A, or S1157V.

13. The method of any of claims 9 to 12, wherein the host further comprises one or more of:
(a) a gene encoding a L-phenylalanine ammonia lyase (PAL) polypeptide;
(b) a gene encoding a cinnamate-4-hydroxylase (C4H) polypeptide;
(c) a gene encoding a NADPH:cytochrome P450 reductase polypeptide;
(d) a gene encoding a tyrosine ammonia lyase (TAL) polypeptide;
(e) a gene encoding a 4-coumarate-CoA ligase (4CL) polypeptide; or
(f) a gene encoding a stilbene synthase (STS) polypeptide;
wherein at least one of said genes is a recombinant gene.

14. The method of any of claims 9 to 13, wherein the stilbene is resveratrol or a resveratrol derivative.

15. The method of any of claims 9 to 14, wherein the recombinant host microorganism comprises *Escherichia* bacteria cells, *Lactobacillus* bacteria cells, *Lactococcus* bacteria cells, *Cornebacterium* bacteria cells, *Acetobacter* bacteria cells, *Acinetobacter* bacteria cells, or *Pseudomonas* bacterial cells.

16. The method any of claims 9 to 14, wherein the recombinant host microorganism is a cell of *Saccharomyces cerevisiae*, *Schizosaccharomyces pombe*, *Yarrowia lipolytica*, *Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous*, or *Candida albicans* species.

## Patentansprüche

1. Rekombinante Wirtsmikroorganismuszelle, die Folgendes umfasst:
(a) ein Gen, das ein Polypeptid der Tyrosin-sensitiven 3-Deoxy-D-arabinoheptulosonat-7-phosphat-Synthase (AR04) kodiert, das mindestens 65 % Identität mit der Aminosäuresequenz aufweist, die in SEQ.-ID Nr. 1 dargelegt ist, wobei das AR04-Polypeptid eine Mutation umfasst, die K229L ist, und keiner Feedback-Inhibition unterliegt;
(b) ein Gen, das ein Chorismat-Mutase-Polypeptid kodiert;
(c) ein Gen, das ein Acetyl-CoA-Carboxylase-Polypeptid kodiert; und
(d) Störung eines Gens, das ein Polypeptid der Phenylalanin-inhibierten 3-Deoxy-D-arabinoheptulosonat-7-phosphat-Synthase (AR03) kodiert, das mindestens 75 % Identität mit der Aminosäuresequenz aufweist, die in SEQ.-ID Nr. 3 dargelegt ist, wodurch der Wirt keine AR03 exprimiert;
wobei mindestens eines der Gene ein rekombinantes Gen ist,
wobei der Wirt ein Stilben produzieren kann.

2. Wirt nach Anspruch 1, wobei das Chorismat-Mutase-Polypeptid ein Chorismat-Mutase (AR07)-Polypeptid umfasst, das mindestens 50 % Identität mit der Aminosäuresequenz aufweist, die in SEQ.-ID Nr. 4 dargelegt ist, wobei das Chorismat-Mutase-Polypeptid ein AR07-Polypeptid umfasst, das eine Mutation aufweist, die T2261 oder T226N ist, und wobei das Chorismat-Mutase-Polypeptid keiner Feedback-Inhibition unterliegt.

3. Wirt nach Anspruch 1 oder 2, wobei das Acetyl-CoA-Carboxylase-Polypeptid ein Acetyl-CoA-Carboxylase-alpha (ACC1)-Polypeptid umfasst, das mindestens 70 % Identität mit der Aminosäuresequenz aufweist, die in SEQ.-ID Nr. 6 dargelegt ist, das eine Mutation an S659 oder S1157 aufweist, wobei die ausgetauschte Aminosäure nicht phosphorylierbar ist und das ACC1-Polypeptid keiner Feedback-Inhibition unterliegt.

4. Wirt nach Anspruch 3, wobei das Acetyl-CoA-Carboxylase-Polypeptid ein ACC1-Polypeptid umfasst, das mindestens eine Mutation aufweist, die S659A, S659V, S1157A oder S1 157V ist.

5. Wirt nach einem vorstehenden Anspruch, der weiter eines oder mehrere von Folgenden umfasst:
(a) ein Gen, das ein L-Phenylalanin-Ammoniak-Lyase (PAL)-Polypeptid kodiert;
(b) ein Gen, das ein Cinnamat-4-Hydroxylase (C4H)-Polypeptid kodiert;
(c) ein Gen, das ein NADPH:Cytochrom-P450-Reduktase-Polypeptid kodiert;
(d) ein Gen, das ein Tyrosin-Ammoniak-Lyase (TAL)-Polypeptid kodiert;
(e) ein Gen, das ein 4-Cumarat-CoA-Ligase (4CL)-Polypeptid kodiert; oder
(f) ein Gen, das ein Stilben-Synthase (STS)-Polypeptid kodiert;
wobei mindestens eines der Gene ein rekombinantes Gen ist.

6. Wirt nach einem der Ansprüche 1-5, wobei das Stilben Resveratrol oder ein Resveratrolderivat ist.

7. Wirt nach Anspruch 6, wobei die rekombinante Wirtsmikroorganismuszelle Folgende umfasst: Escherichia-Bakterienzellen, *Lactobacillus*-Bakterienzellen, *Lactococcus-*Bakterienzellen, *Corynebacterium-*Bakterienzellen, *Acetobacter*-Bakterienzellen*, Acinetobacter-Bakterienzellen* oder *Pseudomonas-Bakterienzellen.*

8. Wirt nach Anspruch 6, wobei die rekombinante Wirtsmikroorganismuszelle eine Zelle aus folgenden Spezies ist: *Saccharomyces cerevisiae*, *Schizosaccharomyces pombe*, *Yarrowia lipolytica, Candida glabrata, Ashbya gossypii, Cyberlindnera jadinii*, *Pichia pastoris, Kluyveromyces lactis, Hansenula polymorpha, Candida boidinii, Arxula adeninivorans*, *Xanthophyllomyces dendrorhous* oder *Candida albicans.*

9. Verfahren zur Produktion eines Stilbens, das Folgendes umfasst:
(a) Züchten eines rekombinanten Wirtsmikroorganismus in einem Kulturmedium unter Bedingungen, unter denen die Gene exprimiert werden,
wobei der Wirt Folgendes umfasst:
(i) ein Gen, das ein Polypeptid der Tyrosin-sensitiven 3-Deoxy-D-arabinoheptulosonat-7-phosphat-Synthase (AR04) kodiert, das mindestens 65 % Identität mit der Aminosäuresequenz aufweist, die in SEQ.-ID Nr. 1 dargelegt ist, wobei das AR04-Polypeptid eine Mutation umfasst, die K229L ist, und keiner Feedback-Inhibition unterliegt;
(ii) ein Gen, das ein Chorismat-Mutase-Polypeptid kodiert;
(iii) ein Gen, das ein Acetyl-CoA-Carboxylase-Polypeptid kodiert; und
(iv) Störung eines Gens, das ein Polypeptid der Phenylalanin-inhibierten 3-Deoxy-D-arabinoheptulosonat-7-phosphat-Synthase (AR03) kodiert, das mindestens 75 % Identität mit der Aminosäuresequenz aufweist, die in SEQ.-ID Nr. 3 dargelegt ist, wodurch der Wirt keine AR03 exprimiert;
wobei mindestens eines der Gene ein rekombinantes Gen ist,
und optional
(b) Gewinnen des Stilbens aus dem Kulturmedium.

10. Verfahren nach Anspruch 9, wobei das Chorismat-Mutase-Polypeptid ein AR07-Polypeptid umfasst, das mindestens 50 % Identität mit der Aminosäuresequenz aufweist, die in SEQ.-ID Nr. 4 dargelegt ist, eine Mutation aufweist, die T2261 oder T226N ist, und wobei das Chorismat-Mutase-Polypeptid keiner Feedback-Inhibition unterliegt.

11. Verfahren nach Anspruch 9 oder 10, wobei das Acetyl-CoA-Carboxylase-Polypeptid ein ACC1-Polypeptid umfasst, das mindestens 70 % Identität mit der Aminosäuresequenz aufweist, die in SEQ.-ID Nr. 6 dargelegt ist, das eine Mutation an S659 oder S1157 aufweist, wobei die ausgetauschte Aminosäure nicht phosphorylierbar ist und das Acetyl-CoA-Carboxylase-Polypeptid keiner Feedback-Inhibition unterliegt.

12. Verfahren nach Anspruch 11, wobei das Acetyl-CoA-Carboxylase-Polypeptid ein ACC1-Polypeptid umfasst, das mindestens eine Mutation aufweist, die S659A, S659V, S1157A oder S1157V ist.

13. Verfahren nach einem der Ansprüche 9 bis 12, wobei der Wirt weiter eines oder mehrere von Folgenden umfasst:
(a) ein Gen, das ein L-Phenylalanin-Ammoniak-Lyase (PAL)-Polypeptid kodiert;
(b) ein Gen, das ein Cinnamat-4-Hydroxylase (C4H)-Polypeptid kodiert;
(c) ein Gen, das ein NADPH:Cytochrom-P450-Reduktase-Polypeptid kodiert;
(d) ein Gen, das ein Tyrosin-Ammoniak-Lyase (TAL)-Polypeptid kodiert;
(e) ein Gen, das ein 4-Cumarat-CoA-Ligase (4CL)-Polypeptid kodiert; oder
(f) ein Gen, das ein Stilben-Synthase (STS)-Polypeptid kodiert;
wobei mindestens eines der Gene ein rekombinantes Gen ist.

14. Verfahren nach einem der Ansprüche 9 bis 13, wobei das Stilben Resveratrol oder ein Resveratrolderivat ist.

15. Verfahren nach einem der Ansprüche 9 bis 14, wobei der rekombinante Wirtsmikroorganismus Folgende umfasst: *Escherichia*-Bakterienzellen, *Lactobacillus-*Bakterienzellen, *Lactococcus-Bakterienzellen*, *Corynebacterium*-Bakterienzellen, *Acetobacter-*Bakterienzellen, *Acinetobacter*-Bakterienzellen oder *Pseudomonas-*Bakterienzellen.

16. Verfahren nach einem der Ansprüche 9 bis 14, wobei der rekombinante Wirtsmikroorganismus eine Zelle aus folgenden Spezies ist: *Saccharomyces cerevisiae, Schizosaccharomyces pombe, Yarrowia lipolytica, Candida glabrata, Ashbya gossypii, Cyberlindnera jadinii*, *Pichia pastoris, Kluyveromyces lactis, Hansenula polymorpha, Candida boidinii*, *Arxula adeninivorans*, *Xanthophyllomyces dendrorhous* oder *Candida albicans.*

## Revendications

1. Cellule de micro-organisme hôte recombiné, comprenant :
(a) un gène codant pour un polypeptide de 3-désoxy-D-arabinoheptulosonate 7-phosphate synthase sensible à la tyrosine (AR04), ayant au moins 65% d'identité par rapport à la séquence d'acides aminés exposée dans SEQ ID NO: 1, où le polypeptide AR04 comprend une mutation qui est K229L et n'est pas inhibé par rétroaction ;
(b) un gène codant pour un polypeptide de chorismate mutase ;
(c) un gène codant pour un polypeptide d'acétyl-CoA carboxylase ; et
(d) la perturbation d'un gène codant pour un polypeptide de 3-désoxy-D-arabinoheptulosonate 7-phosphate synthase inhibé par la phénylalanine (AR03), ayant au moins 75% d'identité par rapport à la séquence d'acides aminés exposée dans SEQ ID NO: 3, ce par quoi l'hôte n'exprime pas AR03 ;
où au moins l'un des gènes est un gène recombiné,
où l'hôte est capable de produire un stilbène.

2. Hôte selon la revendication 1, dans lequel le polypeptide de chorismate mutase comprend un polypeptide de chorismate mutase (AR07) ayant au moins 50% d'identité par rapport à la séquence d'acides aminés exposée dans SEQ ID NO: 4 ; où le polypeptide de chorismate mutase comprend un polypeptide AR07 ayant une mutation qui est T226I ou T226N, et où le polypeptide de chorismate mutase n'est pas inhibé par rétroaction.

3. Hôte selon la revendication 1 ou 2, dans lequel le polypeptide d'acétyl-CoA carboxylase comprend un polypeptide d'acétyl-CoA carboxylase alpha (ACC1) ayant au moins 70% d'identité par rapport à la séquence d'acides aminés exposée dans SEQ ID NO: 6, ayant une mutation au niveau de S659 ou S1157, où l'acide aminé de remplacement est non phosphorylable et le polypeptide ACC1 n'est pas inhibé par rétroaction.

4. Hôte selon la revendication 3, dans lequel le polypeptide d'acétyl-CoA carboxylase comprend un polypeptide ACC1 ayant au moins une mutation qui est S659A, S659V, S1157A, ou S1157V.

5. Hôte selon l'une quelconque des revendications précédentes, comprenant en outre un ou plusieurs parmi :
(a) un gène codant pour un polypeptide de L-phénylalanine ammoniaque lyase (PAL);
(b) un gène codant pour un polypeptide de cinnamate-4-hydroxylase (C4H) ;
(c) un gène codant pour un polypeptide de NADPH:cytochrome P450 réductase ;
(d) un gène codant pour un polypeptide de tyrosine ammoniaque lyase (TAL) ;
(e) un gène codant pour un polypeptide de 4-coumarate-CoA ligase (4CL) ; ou
(f) un gène codant pour un polypeptide de stilbène synthase (STS) ;
où au moins l'un des gènes est un gène recombiné.

6. Hôte selon l'une quelconque des revendications 1-5, dans lequel le stilbène est le resvératrol ou un dérivé du resvératrol.

7. Hôte selon la revendication 6, dans lequel la cellule de micro-organisme hôte recombiné comprend des cellules de bactéries *Escherichia*, des cellules de bactéries *Lactobacillus,* des cellules de bactéries *Lactococcus,* des cellules de bactéries *Corynebacterium*, des cellules de bactéries *Acetobacter,* des cellules de bactéries *Acinetobacter,* ou des cellules de bactéries *Pseudomonas.*

8. Hôte selon la revendication 6, dans lequel la cellule de micro-organisme hôte recombiné est une cellule de *Saccharomyces cerevisiae*, *Schizosaccharomyces pombe*, *Yarrowia lipolytica*, *Candida glabrata*, *Ashbya gossypii*, *Cyberlindnera jadinii*, *Pichia pastoris, Kluyveromyces lactis*, *Hansenula polymorpha*, *Candida boidinii*, *Arxula adeninivorans*, *Xanthophyllomyces dendrorhous* ou *Candida albicans.*

9. Méthode de production d'un stilbène, comprenant :
(a) la culture d'un micro-organisme hôte recombiné dans un milieu de culture, dans des conditions dans lesquelles les gènes sont exprimés,
où l'hôte comprend :
(i) un gène codant pour un polypeptide de 3-désoxy-D-arabinoheptulosonate 7-phosphate synthase sensible à la tyrosine (AR04), ayant au moins 65% d'identité par rapport à la séquence d'acides aminés exposée dans SEQ ID NO: 1, où le polypeptide AR04 comprend une mutation qui est K229L et n'est pas inhibé par rétroaction ;
(ii) un gène codant pour un polypeptide de chorismate mutase ;
(iii) un gène codant pour un polypeptide d'acétyl-CoA carboxylase ; et
(iv) la perturbation d'un gène codant pour un polypeptide de 3-désoxy-D-arabinoheptulosonate 7-phosphate synthase inhibé par la phénylalanine (AR03), ayant au moins 75% d'identité par rapport à la séquence d'acides aminés exposée dans SEQ ID NO: 3, ce par quoi l'hôte n'exprime pas AR03 ;
où au moins l'un des gènes est un gène recombiné,
et, éventuellement
(b) la récupération du stilbène dans le milieu de culture.

10. Méthode selon la revendication 9, dans laquelle le polypeptide de chorismate mutase comprend un polypeptide AR07 ayant au moins 50% d'identité par rapport à la séquence d'acides aminés exposée dans SEQ ID NO: 4, ayant une mutation qui est T226I ou T226N, et où le polypeptide de chorismate mutase n'est pas inhibé par rétroaction.

11. Méthode selon la revendication 9 ou 10, dans laquelle le polypeptide d'acétyl-CoA carboxylase comprend un polypeptide ACC1 ayant au moins 70% d'identité par rapport à la séquence d'acides aminés exposée dans SEQ ID NO: 6, ayant une mutation au niveau de S659 ou S1157, où l'acide aminé de remplacement est non phosphorylable et le polypeptide d'acétyl-CoA carboxylase n'est pas inhibé par rétroaction.

12. Méthode selon la revendication 11, dans laquelle le polypeptide d'acétyl-CoA carboxylase comprend un polypeptide ACC1 ayant au moins une mutation qui est S659A, S659V, S1157A, ou S1157V.

13. Méthode selon l'une quelconque des revendications 9 à 12, dans laquelle l'hôte comprend en outre un ou plusieurs parmi :
(a) un gène codant pour un polypeptide de L-phénylalanine ammoniaque lyase (PAL);
(b) un gène codant pour un polypeptide de cinnamate-4-hydroxylase (C4H) ;
(c) un gène codant pour un polypeptide de NADPH:cytochrome P450 réductase ;
(d) un gène codant pour un polypeptide de tyrosine ammoniaque lyase (TAL) ;
(e) un gène codant pour un polypeptide de 4-coumarate-CoA ligase (4CL) ; ou
(f) un gène codant pour un polypeptide de stilbène synthase (STS) ;
où au moins l'un desdits gènes est un gène recombiné.

14. Méthode selon l'une quelconque des revendications 9 à 13, dans laquelle le stilbène est le resvératrol ou un dérivé de resvératrol.

15. Méthode selon l'une quelconque des revendications 9 à 14, dans laquelle le micro-organisme hôte recombiné comprend des cellules de bactéries *Escherichia*, des cellules de bactéries *Lactobacillus,* des cellules de bactéries *Lactococcus,* des cellules de bactéries *Corynebacterium*, des cellules de bactéries *Acetobacter,* des cellules de bactéries *Acinetobacter,* ou des cellules de bactéries *Pseudomonas.*

16. Méthode selon l'une quelconque des revendications 9 à 14, dans laquelle le micro-organisme hôte recombiné est une cellule de *Saccharomyces cerevisiae, Schizosaccharomyces pombe*, *Yarrowia lipolytica*, *Candida glabrata*, *Ashbya gossypii, Cyberlindnera jadinii, Pichia pastoris, Kluyveromyces lactis*, *Hansenula polymorpha*, *Candida boidinii*, *Arxula adeninivorans*, *Xanthophyllomyces dendrorhous* ou *Candida albicans.*
